(19) Europäisches Patentamt
European Patent Office
Office européen des brevets

(11) **EP 4 071 473 A1**

(12) **EUROPEAN PATENT APPLICATION**
published in accordance with Art. 153(4) EPC


(43) Date of publication:
**12.10.2022 Bulletin 2022/41**

(21) Application number: **20895544.3**

(22) Date of filing: **03.12.2020**

(51) International Patent Classification (IPC):
***G01N 33/50*** *(2006.01)*

(52) Cooperative Patent Classification (CPC):
**A61K 31/4709; A61K 31/675; A61K 31/7068; A61P 35/00; C12Q 1/26; G01N 33/50; G01N 33/88**

(86) International application number:
**PCT/CN2020/133538**

(87) International publication number:
**WO 2021/110085 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.12.2019 CN 201911219742**



(71) Applicant: **Ascentawits Pharmaceuticals, Ltd.**
**Shenzhen, Guangdong 518118 (CN)**

(72) Inventors:
- **DUAN, Jianxin**
  **Shenzhen, Guangdong 518118 (CN)**
- **XIE, Yanbin**
  **Shenzhen, Guangdong 518118 (CN)**
- **JI, Jinfeng**
  **Shenzhen, Guangdong 518118 (CN)**

(74) Representative: **Haseltine Lake Kempner LLP**
**Redcliff Quay**
**120 Redcliff Street**
**Bristol BS1 6HU (GB)**


(54) **METHOD FOR ASSOCIATING WITH EXPRESSION LEVEL OF AKR1C3 ENZYME VIA CONTENT OF PROSTAGLANDIN, AND USE OF SCREENING FOR DRUG ADMINISTRATION**


(57) Disclosed are a method for associating with the expression level of an AKR1C3 enzyme via the content of prostaglandin, and the use of screening for drug administration. In particular, the content of prostaglandin is measured to associate with the expression level of the AKR1C3 enzyme in a biological sample; and the change in the content and the change rate of the content of prostaglandin before and after administering interfering drugs are measured to associate with the expression level of the AKR1C3 enzyme in the biological sample. Also disclosed is an assembly for measuring the expression level of the AKR1C3 enzyme. The assembly includes a component, which reacts with the biological sample by means of coming into contact with same and quantitatively or semi-quantitatively associates with the content of prostaglandin in the biological sample according to a reaction signal; and a control component, which is used to compare the reaction signal to obtain the content of prostaglandin in the biological sample, and the expression level of the AKR1C3 enzyme corresponding to the change in the content and the change rate of the content of prostaglandin before and after administering interfering drugs. Further disclosed is a drug delivery device, which contains an aseembly for measuring the expression lvel of the AKR1C3 enzyme; and an assembly for drug administration containing an AKR1C3 enzyme-activated anti-cancer prodrug.


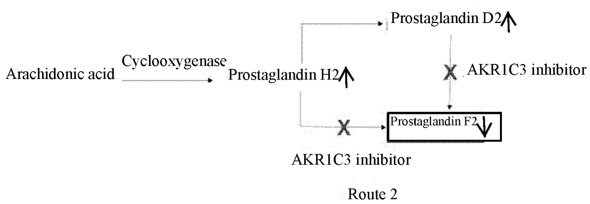


FIG.3

EP 4 071 473 A1

## Description

### Technical Field

The present invention, belonging to the field of research and development of formulation of anti-cancer compounds, relates to the research and development of injection solutions of compounds disclosed in Patent Application No. PCT/US2016/021581 (Publication No. WO2016145092A), which corresponds to Chinese Patent Application No. 2016800150788 (Publication No. CN107530556A); PCT Patent Application No. PCT/US2016/062114 (Publication No. WO2017087428A1), which corresponds to Chinese Patent Application No. 2016800446081 (Publication No. CN108290911A); and PCT Patent Application No. PCT/US2016/025665 (Publication No. WO2016/161342), which corresponds to Chinese Patent Application No. 2016800200132 (Publication No. CN108136214A).

### Background Art

The DNA alkylating anti-cancer prodrug targeting overexpressed aldo-keto reductase 1C3 (AKR1C3) developed by our company (see Patent Applications:

DNA alkylating agents, PCT Patent Application No. PCT/US2016/021581 (Publication No. WO2016/145092A), which corresponds to Chinese Patent Application No. 2016800150788 (Publication No. CN107530556A);

(R)- and (S)-1-(3-(3-N,N-dimethylaminocarbonyl)phenoxy-4-nitrophenyl)-1-ethyl-N,N'-bis (ethylidene)phosphoramidate, compositions and methods of use and preparation thereof, a S configuration compound in PCT Patent Application No. PCT/US2016/062114 (Publication No. WO2017087428A1), which corresponds to Chinese Patent Application No. 2016800446081 (Publication No. CN108290911A);

Nitrobenzyl derivative anti-cancer agents, PCT Patent Application No. PCT/US2016/025665 (Publication No. WO2016/161342), which corresponds to Chinese Patent Application No. 2016800200132 (Publication No. CN108136214A)) is a DNA alkylating anti-cancer prodrug targeting aldo-keto reductase 1C3 (AKR1C3). Compound A in the form of prodrug (i.e., a compound of the general formula I claimed by the invention) is subjected to single-electron reduction under the catalysis of AKR1C3 enzyme in a biochemical environment in cells to obtain an intermediate B which is also unstable and then further reduced under the action of AKR1C3 enzyme to obtain three intermediates C, which are still unstable and are subjected to 1,4 elimination reaction to finally obtain

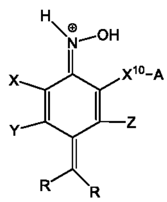

;

and cytotoxic H-$L^1$-D, which plays a role in killing cancer cells, as shown in Figure 1: The schematic diagram of the metabolic mechanism of DNA alkylating anti-cancer prodrugs targeting aldo-keto reductase 1C3 (AKR1C3), (the above schematic diagram Figure 1 is drawn according to the following documents:

Document 1: Kathryn Evans, JianXinDuan,Tara Pritchard,et al.OBI-3424,a novel AKR1C3-activated prodrug,exhibits potent efficacy against preclinical models of T-ALL[J], ClinicalCancerResearch,2019,DOI:10.1158/1078-0432.CCR-19-0551;

Document 2: Richard B.Lock,Kathryn Evans,Raymond Yung et al.Abstract LB-B16:The AKR1C3-Activated Prodrug OBI-3424 Exerts Profound InVivo Efficacy Against Preclinical Models of T-Cell Acute Lymphoblastic Leukemia(T-ALL);a Pediatric Preclinical Testing Consortium Study[C], AACR-NCI-EORTC International Conference:Molecular Targetsand Cancer Therapeutics;October 26-30,2017; Philadelphia,PA, DOI:10.1158/1535-7163;

Document 3: Jianxin Duan, Zhong Wang, Qing Li et al.Broad In Vitro and In Vivo Antitumor Activity of TH-3424: Preclinical Rationale for a Highly Selective AKR1C3 Prodrug for Treating Cancer, AACR Annual Meeting 2016, Abstract #1369, April 16-20,2016; New Orleans, LA).

Specifically, the compound with a name of (S)-1-(3-(3-N,N-dimethylaminocarbonyl)phenoxy-4-nitrophenyl)-1-ethyl-N,N' -bis (ethylidene)phosphoramidate, also known as a S configuration compound of OBI-3424 and TH-2870 (CAS No. 2097713-69-2), has the following structure:

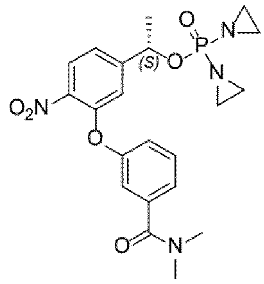

Chemical Structural Formula of AST-3424

A Phase I clinical trial has been conducted in the United States and China, respectively.

According to Document 3 and the mechanism of drug, it is known that the drug is only effective for patients with AKR1C3 expression, so it is necessary to detect the expression level of AKR1C3 in patients.

The conventional detection method is to directly obtain tumor tissue sections from the patients (Document 4: Christopher P. Guise, Maria R. Abbattista, Rachelle S. Singleton, et al, The Bioreductive Prodrug PR-104A Is Activated under Aerobic Conditions by Human Aldo-Keto Reductase 1C3, Cancer Res, 70 (4): 1573-1584; DOI:10.1158/0008-5472.CAN-09-3237), and then use an immunohistochemical staining method (IHC) for dectection.

However, tumor tissue sections cannot be obtained under certain occasions and conditions: tumor tissue sections of certain patients are lost so that they cannot be or are inconvenient to be obtained again (tumor tissue sections of certain patients are lost due to improper storage); certain patients are unwilling to accept the section sampling operation (the sections are obtained from the body part of the patient by forceps, excision or puncture aspiration and the like according to different situations) due to culture, concept or religious habit and the like; the patients with certain cancers or tumors cannot be sectioned.

## Summary of the Invention

The invention provides a novel detection method which can be adapted to a plurality of biological samples to detect the expression level of associated AKR1C3.

The research and development group conceived that since the compounds disclosed in the above three applications for invention serve as specific substrates of aldo-keto reductase AKR1C3 (hereinafter abbreviated as specific substrates), which are specifically activated by the aldo-keto reductase AKR1C3 and metabolized to obtain cytotoxic alkylating agents, that is, the aldo-keto reductase AKR1C3 needs to bind to the specific substrate to function.

According to research documents (Document 5: Samad T A, Moore K A, Sapirstein A, et al. Interleukin-1[beta]-mediated induction of Cox-2 in the CNS contributes to inflammatory pain hypersensitivity[J]. Nature, 2001, 410(6827):471-5.;

Document 6: Baojian Z , Yanbing Y , Gele A , et al. Tanshinone IIA Attenuates Diabetic Peripheral Neuropathic Pain in Experimental Rats via Inhibiting Inflammation[J]. Evidence-Based Complementary and Alternative Medicine, 2018, 2018:1-8.;

Document 7: Lovering A, Ride J , Bunce C , et al. Crystal Structures of Prostaglandin D2 11-Ketoreductase (AKR1C3) in Complex with the Nonsteroidal Anti-Inflammatory Drugs Flufenamic Acid and Indomethacin[J]. Cancer Research, 2004, 64(5):1802-1810.;

Document 8: Matsuura K, Shiraishi H, Hara A, et al. Identification of a principal mRNA species for human 3alpha-hydroxysteroid dehydrogenase isoform (AKR1C3) that exhibits high prostaglandin D2 11-ketoreductase activity.[J]. Journal of Biochemistry, 1998, 124(5):940-6.), it is known that aldo-keto reductase AKR1C3 plays an important catalytic role in the biochemical pathway of the conversion of prostaglandin H2/D2 to prostaglandin E2/F2 (prostaglandin F2 is also known as prostaglandin F2$\alpha$), as shown in Figure 2: The schematic diagram of the effect of AKR1C3 on prostaglandin metabolism.

The inventors envisaged that since AKR1C3 plays a role in the conversion of prostaglandins, whether the content change of prostaglandin F2 (a product of the conversion process) or prostaglandin H2/D2 (a reactant of the conversion process), can be used to associate with expression level of AKR1C3. Specifically, whether there are the following corresponding relationships:
Before and after administration of the inhibitor or agonist of AKR1C3, whether the corresponding content change of product prostaglandin F2 before and after the conversion process or reactant prostaglandin H2/D2 in the conversion process indicates that a large change means that the expression level of AKR1C3 is high (as shown in Figure 3: The schematic diagram of the effect of AKR1C3 inhibitor on prostaglandin metabolism), and a small change means that the expression level of AKR1C3 is low (as shown in Figure 4: The schematic diagram of the effect of AKR1C3 agonist on prostaglandin metabolism).

The rationality of the above idea can be verified through *in vivo* animal experiments, and the above guess and reasoning can be verified through analysis of experimental results.

The following technical schemes are provided for this purpose.

Scheme 1: A method for measuring expression level of AKR1C3 enzyme

The present invention provides a method for measuring the expression level of AKR1C3 enzyme, comprising measuring the content of PGF2α and/or PGD2 and/or PGH2 to associate with the expression level of AKR1C3 enzyme in a biological sample.

The expression level of AKR1C3 enzyme is generally evaluated by non-expression (undetectable), low, medium and high. For example, according to the description in Document 4, the grading is determined according to the degree of staining after staining using IHC method: diffuse, determined to be highly expressed when all cells are stained; moderate, determined to be mediumly expressed when most or many cells are stained; few, determined to be lowly expressed when only a few cells are stained; and non-expression, determined to be negative when staining was not observed.

Of course, high, medium and low grades can also be determined according to the percentage of the stained area to the overall area after the sample is subjected to IHC staining.

In this scheme, the determination is made directly by measuring the content of PGF2α and/or PGD2 and/or PGH2:
A large number of sample statistics were subjected to generate an association formula between the content of PGF2α and/or PGD2 and/or PGH2 in the population and the expression level of AKR1C3 enzyme (the expression level herein is quantified by the percentage of the stained area to the overall area after IHC staining). That is, when the content of PGF2α and/or PGD2 and/or PGH2 in a person is greater than the high value H, the expression level of AKR1C3 enzyme could be directly considered to be high within a certain confidence range; and when the content of PGF2α and/or PGD2 and/or PGH2 in a person is lower than the low value L, the expression level of AKR1C3 enzyme could be directly considered to be low within a certain confidence range, while the remaining ones between the high value H and the low value L are determined as medium expression.

Further, in the above scheme, the association refers to:

when the content of PGF2α is in the range of A1, the expression level of AKR1C3 enzyme in the biological sample is high;

when the content of PGF2α is in the range of B1, the expression level of AKR1C3 enzyme in the biological sample is medium;

when the content of PGF2α is in the range of C1, the expression level of AKR1C3 enzyme in the biological sample is low;

and/or

when the content of PGD2 and/or PGH2 is in the range of A2, the expression level of AKR1C3 enzyme in the biological sample is low;

when the content of PGD2 and/or PGH2 is in the range of B2, the expression level of AKR1C3 enzyme in the biological sample is medium;

when that content of PGD2 and/or PGH2 is in the range of C2, the expression level of AKR1C3 enzyme in the biological sample is high,

wherein the minimum value in the range of A1 is greater than or equal to the maximum value in the range of B1, the minimum value in the range of B1 is greater than or equal to the maximum value in the range of C1, i.e., the range of A1 is a (m, n) interval, the range of B1 is a (p, q) interval, and the range of C1 is a (r, s) interval, then the minimum value n in the range of A1 is greater than or equal to the maximum value p in the range of B1, and the minimum value q in the range of B1 is greater than or equal to the maximum value r in the range of C1. The units for the six ranges A1, B1, C1, and A2, B2, C2 are the units of content (mass/volume) and pg/ml in human samples.

The minimum value in the range of A2 is greater than or equal to the maximum value in the range of B2, and the minimum value in the range of B2 is greater than or equal to the maximum value in the range of C2.

Obviously, the above direct determination method is the most rapid and convenient.

Further, considering that prostaglandin F2 in roadmap 1/2/3 is the final product of the metabolic pathway, and its content is directly related to AKR1C3, while the metabolism of prostaglandin D2/H2 is also affected by other factors (such as there are other metabolic pathways), therefore, the expression level of AKR1C3 is not the only factor for determining prostaglandin D2/H2. Therefore, the method for measuring the content of prostaglandin F2 is the preferred one among the above three schemes.

Since it is necessary to determine six ranges: A1, B1, C1, and A2, B2, C2, respectively, which are obtained by smoke statistics of a population, there may be a problem that a conclusion of a statistical sample may not necessarily represent a certain specific subject: in fact, the case that a certain sample just falls outside the statistical sample cannot be ruled out, and the statistic and association of a large number of samples are also a time-consuming and resource-consuming work, and more importantly, differences in race and living region may render that the result of the method is inaccurate or applicable population is narrow.

To this end, this scheme further proposes a method using drugs for interference: a method for measuring the expression level of AKR1C3 enzyme, comprising measuring the content change of PGF2$\alpha$ and/or PGD2 and/or PGH2 before and after administration of the interference drug to assocaite with the expression level of AKR1C3 enzyme in a biological sample.

The above method comprises associate with the expression level of AKR1C3 enzyme by obtaining the content change of PGF2$\alpha$ and/or PGD2 and/or PGH2 before and after administration of the interference drug. Similarly, a large number of sample statistics are subjected to generate a association formula between the content change of PGF2$\alpha$ and/or PGD2 and/or PGH2 before and after administration of the same interference drug in the population and the expression level of AKR1C3 enzyme (the expression level herein is quantified by the percentage of the stained area to the overall area after IHC staining), that is, when the content change of PGF2$\alpha$ and/or PGD2 and/or PGH2 before and after administration of a certain interference drug in a person is greater than the high value H, the expression level of AKR1C3 enzyme can be directly considered as high within a certain confidence range; and when the content change of PGF2$\alpha$ and/or PGD2 and/or PGH2 before and after administration of a certain interference drug in a person is lower than the low value L, the expression level of AKR1C3 enzyme can be directly considered as low within a certain confidence range, while the remaining ones between the high value H and the low value L are determined as medium expression.

Based on this method, different datas of different interference drugs (different AKR1C3 inhibitors and different AKR1C3 agonists) can be obtained statistically to verify each other, so that the influence caused by the difference of race and living region can be reduced or weakened to a certain extent, and conclusions obtained from smaller samples can be adapted to a wider population.

Further, in the above scheme, the association refers to:

when the content change of PGF2$\alpha$ is in the range of a1, the expression level of AKR1C3 enzyme in the biological sample is high;

when the content change of PGF2$\alpha$ and/or PGD2 and/or PGH2 is in the range of b1, the expression level of AKR1C3 enzyme in the biological sample is medium;

when the content change of PGF2$\alpha$ and/or PGD2 and/or PGH2 is in the range of c1, the expression level of AKR1C3 enzyme in the biological sample is low;

and/or

when the content change of PGD2 and/or PGH2 is in the range of a2, the expression level of AKR1C3 enzyme in the biological sample is low;

when the content change of PGD2 and/or PGH2 is in the range of b2, the expression level of AKR1C3 enzyme in the biological sample is medium;

when the content change of PGD2 and/or PGH2 is in the range of c2, the expression level of AKR1C3 enzyme in the biological sample is high,

wherein the minimum value in the range of a1 is greater than or equal to the maximum value in the range of b1, and the minimum value in the range of b1 is greater than or equal to the maximum value in the range of c1, i.e.

the minimum value in the range of a2 is greater than or equal to the maximum value in the range of b2, and the minimum value in the range of b2 is greater than or equal to the maximum value in the range of c2.

Further, considering that prostaglandin F2 in roadmap 1/2/3 is the final product of the metabolic pathway, and its content is directly related to AKR1C3, while the metabolism of prostaglandin D2/H2 is also affected by other factors (such as there are other metabolic pathways), therefore, the expression level of AKR1C3 is not the only factor for determining prostaglandin D2/H2. Therefore, the method for measuring the content change of prostaglandin F2 before and after administration of the interference drug is the preferred one among the above three schemes.

Furthermore, in the above method for performing association using absolute change, there may be a case where the absolute value of the content change of PGF2α and/or PGD2 and/or PGH2 in the statistical sample is large, so that a certain value in the statistical sample is too far from the center value (high, medium, and low expressions each correspond to three center values), and thus the change rate is introduced to overcome this problem.

The present invention provides a method for measuring the expression level of AKR1C3 enzyme, comprising measuring the content change rate of PGF2α and/or PGD2 and/or PGH2 before and after appliation of the interference drug to associate with the expression level of AKR1C3 enzyme in the biological sample. Similarly, a large number of sample statistics are subjected to generate an association formula between the content change rate of PGF2α and/or PGD2 and/or PGH2 before and after administration of the same interference drug in the population (defined as the percentage of the content change to the content before drug applcation) and the expression level of AKR1C3 enzyme (the expression level herein is quantified by the percentage of the stained area to the overall area after IHC staining), that is, when the content change rate of PGF2α and/or PGD2 and/or PGH2 before and after administration of a certain interference drug in a person is greater than the high value H, the expression level of AKR1C3 enzyme can be directly considered as high within a certain confidence range; and when the content change rate of PGF2α and/or PGD2 and/or PGH2 before and after administration of a certain interference drug in a person is lower than the low value L, the expression level of AKR1C3 enzyme can be directly considered as low within a certain confidence range, while the remaining ones between the high value H and the low value L are determined as medium expression.

Based on the method, different datas of different interference drugs (different AKR1C3 inhibitors and different AKR1C3 agonists) can be obtained statistically to verify each other, and the concept of change rate is introduced, so that the effect of the absolute content of PGF2α and/or PGD2 and/or PGH2 in an individual is weakened to a certain extent, and the association between the change rate and the expression level of AKR1C3 is highlighted; therefore, the influncence caused by difference of race and living region can be better alleviated and weakened; and conclusions obtained by sample statistical analysis can be applied to a wider population and have a more accurate association result.

Furthermore, in the above scheme, the association refers to:

when the content change rate of PGF2α is in the range of α1, the expression level of AKR1C3 enzyme in the biological sample is high;

when the content change rate of PGF2α is in the range of β1, the expression level of AKR1C3 enzyme in the biological sample is medium;

when the content change rate of PGF2α is in the range of yl, the expression level of AKR1C3 enzyme in the biological sample is low,

and/or

when the content change rate of PGD2 and/or PGH2 is in the range of α2, the expression level of AKR1C3 enzyme in the biological sample is low;

when the content change rate of PGD2 and/or PGH2 is in the range of β2, the expression level of AKR1C3 enzyme in the biological sample is medium;

when the content change rate of PGD2 and/or PGH2 is in the range of γ2, the expression level of AKR1C3 enzyme in the biological sample is high,

wherein the minimum value in the range of α1 is greater than or equal to the maximum value in the range of β1, and the minimum value in the range of β1 is greater than or equal to the maximum value in the range of γ1,

the minimum value in the range of α2 is greater than or equal to the maximum value in the range of β2, and the minimum in the range of β2 is greater than or equal to the maximum value in the range of γ2.

Further, considering that prostaglandin F2 in roadmap 1/2/3 is the final product of the metabolic pathway, and its content is directly related to AKR1C3, while the metabolism of prostaglandin D2/H2 is also affected by other factors (such as there are other metabolic pathways), therefore, the expression level of AKR1C3 is not the only factor for determining prostaglandin D2/H2. Therefore, the method for measuring the content change rate of prostaglandin F2 before and after administration of the interference drug is the preferred one among the above three schemes.
Further, the interference drug is an AKR1C3 enzyme inhibitor or an AKR1C3 enzyme agonist.
Known AKR1C3 enzyme inhibitors include those disclosed in the following Patent Applications:

| Publication No. | The name of Patent Application | Application No. |
|---|---|---|
| US20130116277A1 | Aldo-keto reductase subfamily 1c3 (akrlc3) inhibitors | US13607633 |
| US20180305305A1 | 2-beta-naphthyl-acetic acid analogs as akrlc3 inhibitors and methods of using same | US15769565 |
| US20160159731A1 | Bifunctional akrlc3 inhibitors/androgen receptor modulators and methods of use thereof | US14993742 |
| US20140107085A1 | Bifunctional akrlc3 inhibitors/androgen receptor modulators and methods of use thereof | US14050937 |
| US20170260226A1 | 3-nitrogen or sulphur substituted oestra-1,3,5(10),16-tetraene akrlc3 inhibitors | US15510348 |
| US20160303082A1 | Indomethacin analogs for the treatment of castrate-resistant prostate cancer | US15132937 |
| US20180271835A1 | Indomethacin analogs for the treatment of castrate-resistant prostate cancer | US15899171 |
| US20140371261A1 | Indomethacin analogs for the treatment of castrate-resistant prostate cancer | US14352421 |
| US20140249119A1 | Estra-1,3,5(10),16-tetraene-3-carboxamide derivatives, processes for their preparation, pharmaceutical preparations comprising them and their use for preparing medicaments | US14348645 |
| US20150210734A1 | 3-substituted estra-1,3,5(10),16-tetraene derivatives, methods for the production thereof, pharmaceutical preparations containing same, and use thereof for the production of medicaments | US14414386 |
| US20160024142A1 | Estra-1,3,5(10),16-tetraene-3-carboxamides for inhibition of 17.beta.-hydroxysteroid dehydrogenase (akr1 c3) | US14770444 |
| US20090275608A1 | Methods of diagnosing and treating parp-mediated diseases | US12322551 |
| US20170342082A1 | [8-(phenylsulfonyl)-3,8-diazabicyclo[3.2.1]oct-3-yl](1h-1 ,2,3-triazol-4-yl)methanones | US15596383 |
| US20180319807A2 | [8-(phenylsulfonyl)-3,8-diazabicyclo[3.2.1]oct-3-yl](1h-1 ,2,3-triazol-4-yl)methanones | US15596383 |
| CN201811133143.4 | A dihydropyranopyrazole compound, preparation method and application thereof | CN109305972A |

and indomethacin and other Chinese herbal medicines, of course, TH-2870 and the like.

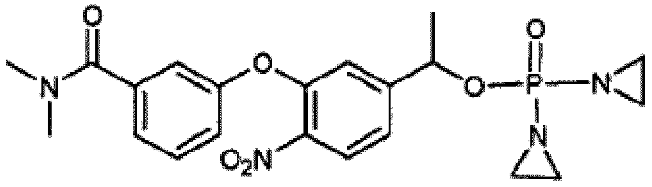

Chemical Structural Formula of TH2870

An agonist is a chemical that binds to and activates a receptor to produce a biological reaction. Agonists cause the effect, while antagonists block the effect of the agonist, and inverse agonists cause the effect opposite to the agonist.

Preferably, the AKR1C3 enzyme inhibitor is

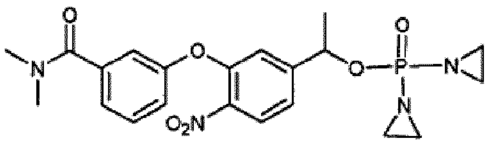 ; or 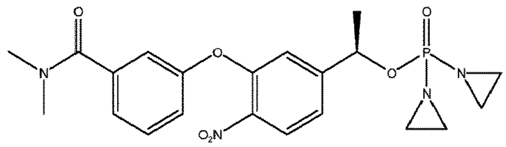 ;

or

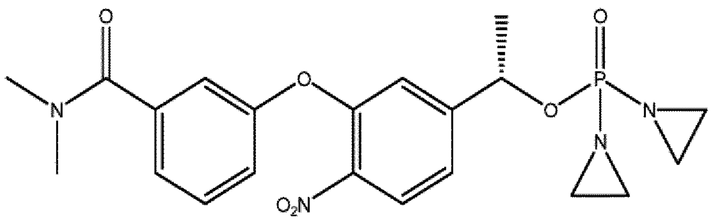

; or Indomethacin.

Among the above preferred compounds,

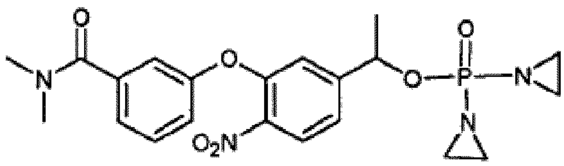

and the isomers thereof in R/S configuration are compounds with AKR1C3 inhibitory activity verified by experiments, while indomethacin is a drug that has been marketed.

Those skilled in the art can select different forms of biological samples according to the situation, including blood or serum, tissues or skin or tumor tissue sections, and perform dectection according to different situations. In contrast, since blood is readily available, it is preferable that the biological sample (biological specimen) is blood.

There are relatively mature methods for detecting the content of prostaglandins (PGF2$\alpha$ and/or PGD2 and/or PGH2) in blood. For example, there are commercial kits and related methods for enzyme-linked immunosorbent assay (ELISA) kits for detecting PGF2$\alpha$.

The present invention provides a measuring method, comprising the following operations:
Operation I. Measurement of the content of PGF2a and/or PGD2 and/or PGH2 in a living body, a living biological organ or a living biological tissue.

Obviously, if we have a reliable (with high enough confidence level) statistical data conclusion that matches the living region and race corresponding to the measured living body or living biological organ or living biological tissue subject, we can obtain the expression level of AKR1C3 corresponding to the living body or organ or tissue according to the corresponding association relationship through Operation I.

If there are no suitable statistical data conclusions, the following interference operations are required:

Operation II. Administration of the interference drug to the living body, the living biological organ or the living biological tissue.

The administration of the interference drug herein needs to be adjusted according to different situations: the living body (animal or human) is administered with the interference drug: AKR1C3 inhibitors or agonists by oral or injection administration (preferably oral administration, with a faster effect); for the living biological organ, either infusion or injection administration can be performed, and the living biological tissue can only be immersed in a solution of AKR1C3 inhibitor or agonist (containing appropriate buffer components) for incubation reaction.

Of course, different administration methods have different optimal measurement times: in general, the amount of drug to be applied or the concentration of exposure time (for tissues subjected to immersion incubation) in different living boides or different organs and tissues thereof should be experimentally explored to obtain the optimal amount of drug to be applied or the optimal exposure time as well as the time for sampling and testing again after administration.

Operation III. Measurement of the content of PGF2α and/or PGD2 and/or PGH2 in the living body, the living biological organ or the living biological tissue after administration of the interference drug.

Obviously, the specific time "after administration of the interference drug" needs to be experimentally explored, which is related to the half-life $T_{1/2}$ of the interference drug for administration to the living body.

Operation IV. Calculation of the content change and the content change rate before and after administration of the interference drug, and obtaining the expression level of the AKR1C3 enzyme in the biological sample according to the corresponding relationship.

Scheme 2: A method for screening and administering

A method for screening and administering to a patient with cancer, tumor, or a disorder caused by cancer or tumor, or a cell proliferative disease comprises administering an AKR1C3 enzyme-activated anti-cancer prodrug to the patient after the expression level of AKR1C3 enzyme is obtained by using the above method for measuring the expression level of AKR1C3 enzyme.

In the present invention, the AKR1C3 enzyme-activated anti-cancer prodrugs, i.e., compounds in the form of prodrugs, are activated by catalysis of AKR1C3 enzymes in the biochemical environment in cells to finally obtain a cytotoxic toxin to play a role in killing cancer cell. See the compounds disclosed in the following patent applications:

PCT/US2016/021581 (Publication No. WO2016145092A1), which corresponds to Chinese Patent Application No. 2016800150788 (Publication No. CN107530556A);

PCT/US2016/025665 (Publication No. WO2016161342), which corresponds to Chinese Patent Application No. 2016800446081 (Publication No. CN108290911A);

PCT/US2016/062114 (Publication No. WO2017087428), which corresponds to Chinese Patent Application No. 2016800200132 (Publication No. CN108136214A); and

PCT/NZ2019/050030 (Publication No. WO2019190331), which corresponds to Chinese Patent Application No.201980023423.6 (Publication No. CN111918864A).

The compounds of the general formula and specific compounds disclosed in the above patent applications all belong to AKR1C3-activated anti-cancer prodrugs (compounds), and the above applications are herein incorporated into the description of the present application.

A method for screening and administering to a patient with cancer, tumor, or a disorder caused by cancer or tumor, or a cell proliferative disease comprises administering an AKR1C3 enzyme-activated anti-cancer prodrug to the patient when the level of enzyme is high after the expression level of AKR1C3 enzyme is obtained from using the above method for measuring the expression level of AKR1C3 enzyme.

A method for screening and administering to a patient with cancer, tumor, or a disorder caused by cancer or tumor, or a cell proliferative disease comprises administering an AKR1C3 enzyme-activated anti-cancer prodrug to the patient when the enzyme level is high or medium after the expression level of AKR1C3 enzyme is obtained from using the above method for measuring the expression level of AKR1C3 enzyme.

Further, in the above administration method, according to the compounds disclosed in Patent Application No. PCT/US2016/021581 (Publication No. WO2016145092A1), which corresponds to the Chinese Patent Application No. 2016800150788 (Publication No. CN107530556A), the AKR1C3 enzyme-activated anti-cancer prodrug comprises the compound of structural formula I:

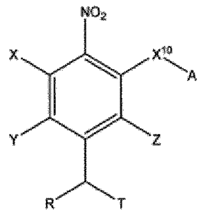

I

wherein,

$X^{10}$ is O, S, SO or $SO_2$;

A is $C_6$-$C_{10}$ aryl or substituted aryl, 5-15 membered heteroaryl or substituted heteroaryl or -N = $CR^1R^2$; wherein $R^1$ and $R^2$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

X, Y and Z are each independently hydrogen, CN, halogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

$R^{13}$ and $R^{14}$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl or ether, or the $R^{13}$ and $R^{14}$ groups together with the nitrogen atom to which they are bonded form 5-7 membered heterocyclyl;

T comprises an amino phosphate alkylating agent, i.e., T is -L-D, and includes the following six cases:

-D is -$P(Z^1)(Z^5$-$X^5$-$Y^5)_n$, $Z^1$ is O or S, $Z^5$ is N, S or O, $X^5$ is an optionally substituted ethylidene, $Y^5$ is a halogen atom or -$OSO_2$-$R^{20}$, $R^{20}$ is an optionally substituted hydrocarbyl, aryl, cycloalkyl, heterocyclyl or heteroaryl, n is 1 or 2, and L is selected from -O-, -S-, -OCOO-, -$NR^6CO$-, -OCO-, -$NR^6SO_2$-, -$OCONR^6$-, quaternary ammonium, sulfonate group-$OSO_2$-;

or

$Z^1$ is O or S, and $Z^5$-$X^5$-$Y^5$ is an aziridinyl -$NCH_2CH_2$ moiety;

or

-L- is -O-, -D is -$P(Z^1)(Z^5$-$X^5$-$Y^5)_n$, $Z^1$ is O or S, $Z^5$ is N, S or O, $X^5$ is an optionally substituted ethylidene, $Y^5$ is a halogen atom or -$OSO_2$-$R^{20}$, $R^{20}$ is an optionally substituted hydrocarbyl, aryl, cycloalkyl, heterocyclyl or heteroaryl, and n is 1 or 2;

or

-L- is -O-, $Z^1$ is O or S, and $Z^5$-$X^5$-$Y^5$ is an aziridinyl -$NCH_2CH_2$ moiety;

or

-L-D is -$OP(Z^1)(NR^{30}CH_2CH_2Cl)_2$, -$OP(Z^1)(NR^{30}CH_2CH_2Br)_2$, -$OP(Z^1)(NR^{30}_2)(N(CH_2CH_2X^1)_2)$, -$OP(Z^1)(N(CH_2)_2)_2$, or -$OP(Z^1)(N(CH_2CH_2Cl)_2)_2$, wherein each $R^{30}$ is independently H, $C_1$-$C_6$ hydrocarbyl or two $R^{30}$ groups together with the nitrogen atom to which they are linked form 5-7 membered heterocycle, $Z^1$ is O or S, and $X^1$ is Cl, Br or -$OSO_2Me$;

or

-L-D is -$OP(Z^1)(NHCH_2CH_2Cl)_2$, -$OP(Z^1)(NHCH_2CH_2Br)_2$, -$OP(Z^1)(NH_2)(N(CH_2CH_2X^1)_2)$, -$OP(Z^1)(N(CH_2)_2)_2$

or -OP($Z^1$)(N($CH_2CH_2Cl$)$_2$)$_2$, and $X^1$ is Cl, Br, or -$OSO_2Me$;

and the alkyl, alkenyl, alkynyl, cycloalkyl, aryl, heterocycle, heteroaryl and the ether group are substituted or unsubstituted.

The substituent is a halogen atom, cyano or isocyano, thiocyano or isothiocyano, hydroxyl, mercapto, amino, oximido, a hydrazone group, OTs, OMS, $C_1$-$C_3$ alkyl or substituted alkyl, $C_1$-$C_3$ alkoxy or substituted alkoxy, $C_2$-$C_3$ alkenyl or substituted alkenyl, $C_2$-$C_3$ alkynyl or substituted alkynyl, $C_3$-$C_8$ cycloalkyl or substituted cycloalkyl, an aromatic ring, heterocycle, a heteroaromatic ring and a fused ring or a substituted aromatic ring, heterocycle, a heteroaromatic ring and a fused ring, wherein the substituted mode is monosubstituted or geminal disubstituted, and the substituent is a halogen atom, cyano or isocyano, thiocyano or isothiocyano, hydroxyl, mercapto, amino, oximido, a hydrazone group, OTs or OMs.

Further, the AKR1C3 enzyme-activated anti-cancer prodrug contains a compound having a structural formula selected from the group consisting of:

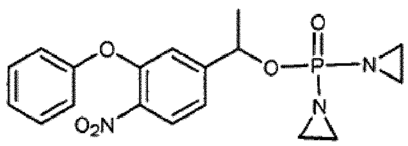,
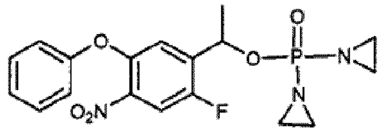,

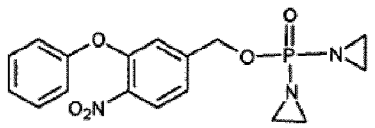,
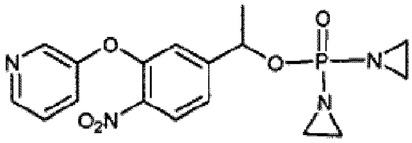,

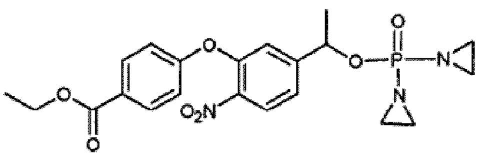,
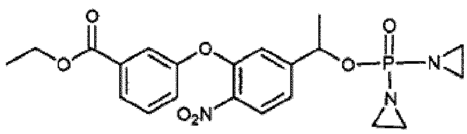,

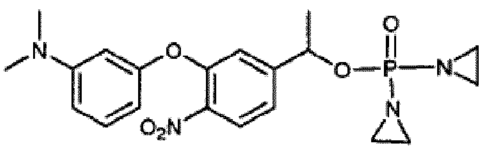,
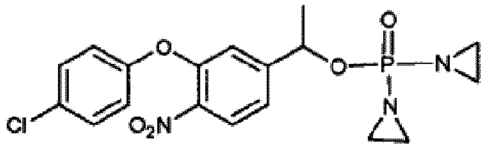,

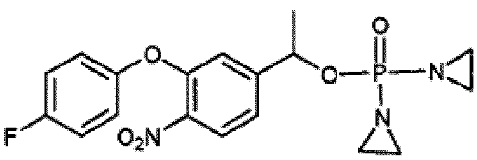,
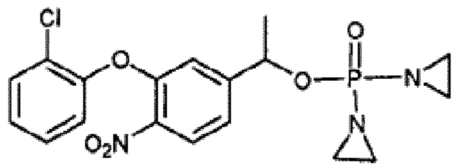,

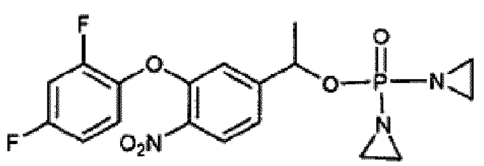,
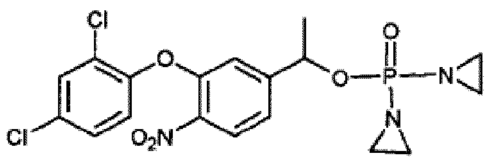,

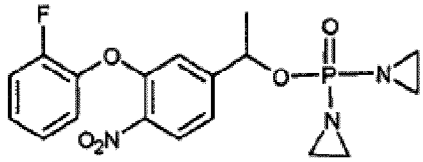,
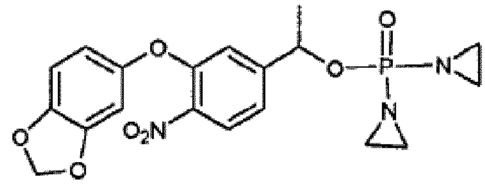,
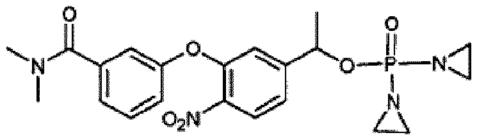,
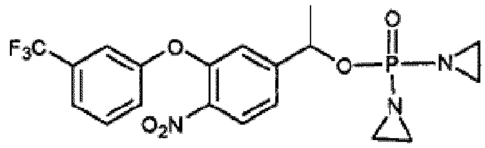,
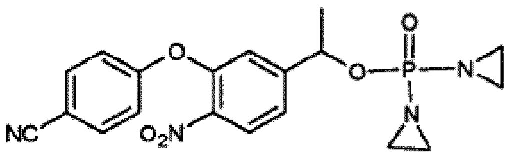,
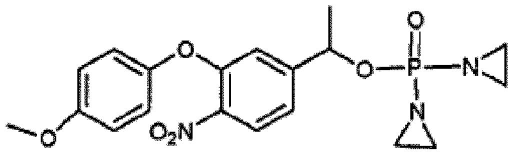,
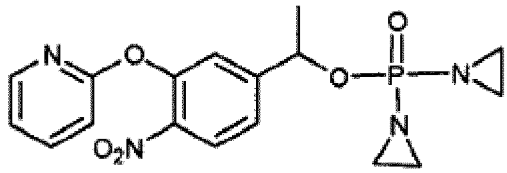,
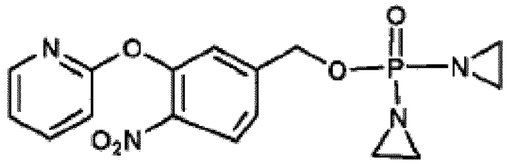,
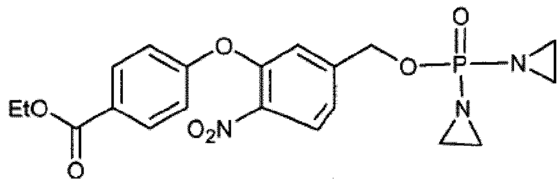,
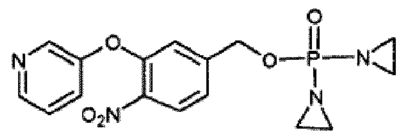,
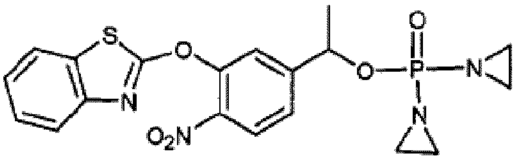,
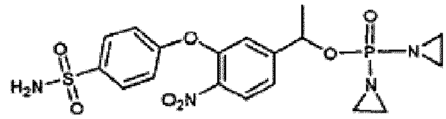,
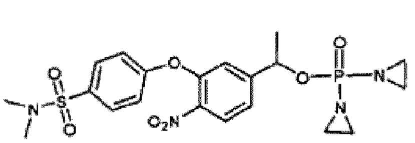,
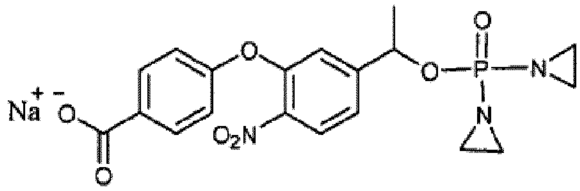,

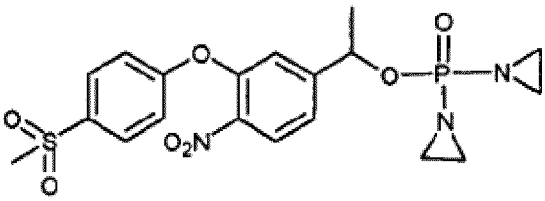
,
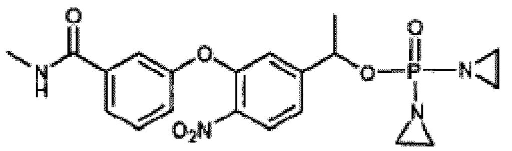
,

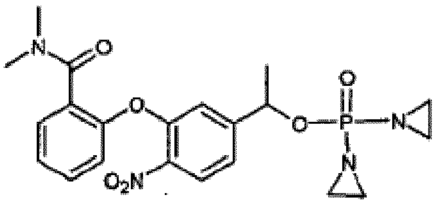
,
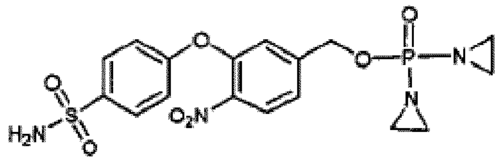
,

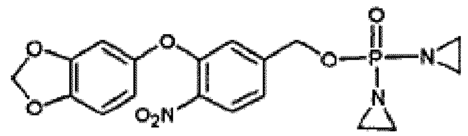
,
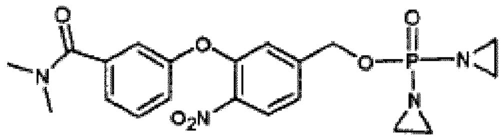
,

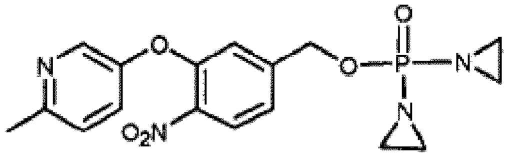
,
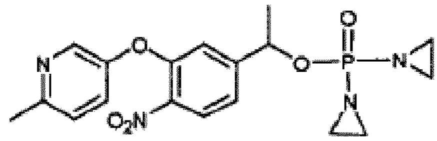
,

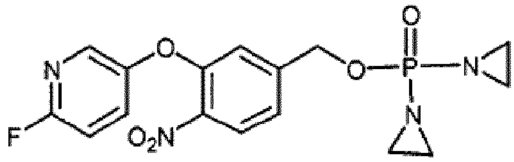
,
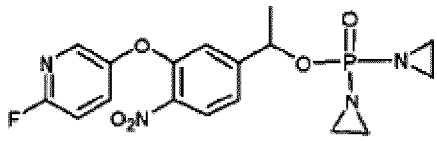
,

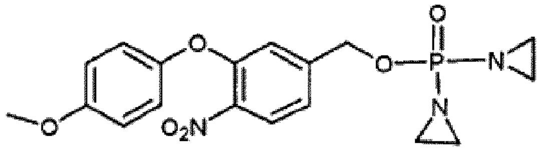
,
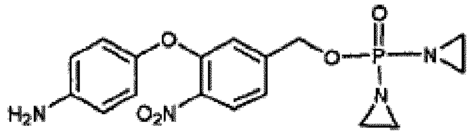
,

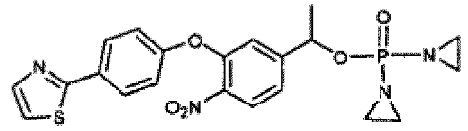
,
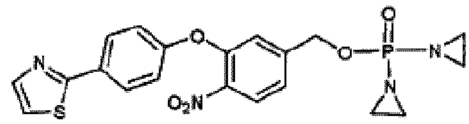
,

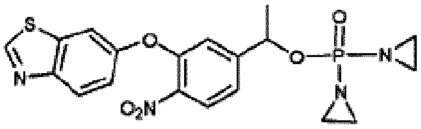
,
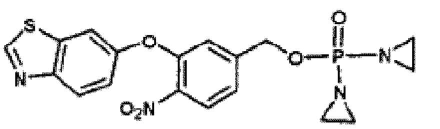
,

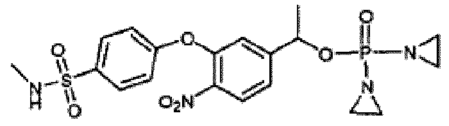 , 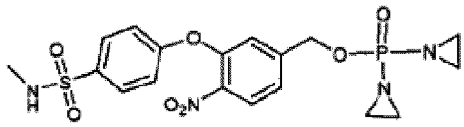 ,

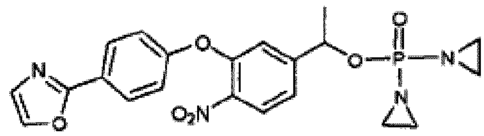 , 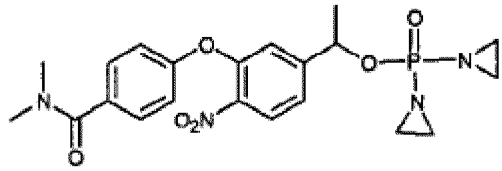 ,

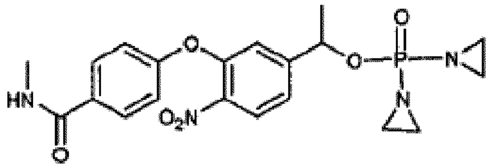 , 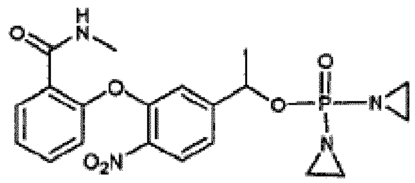 ,

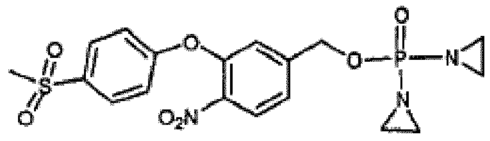 , 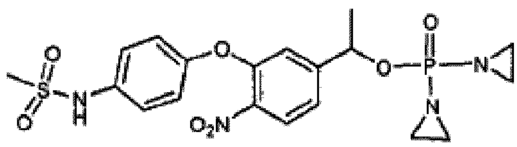 ,

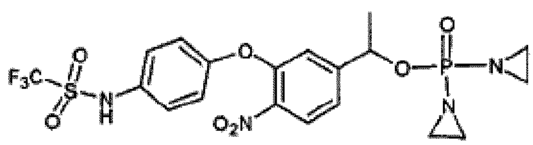 , 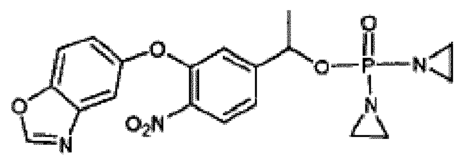 ,

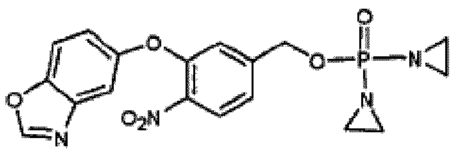 , 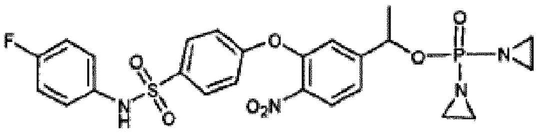 ,

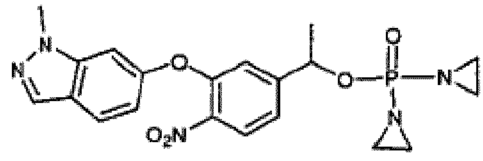 , 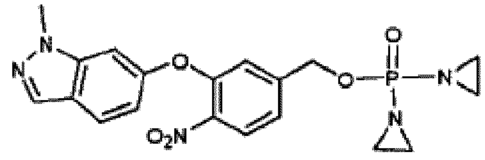 ,

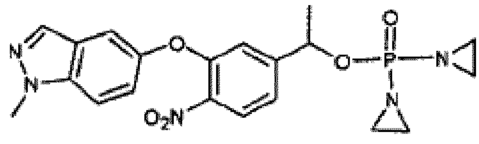 , 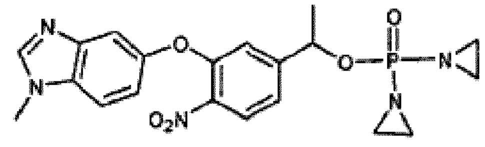 ,

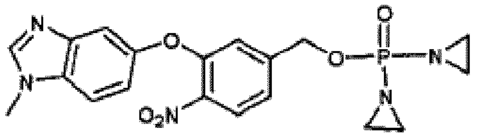,
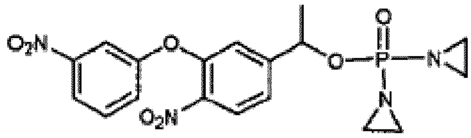,

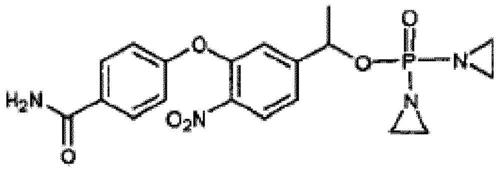,
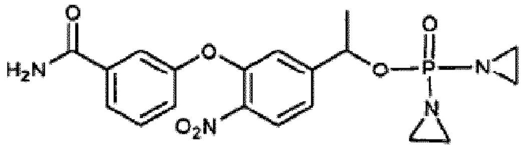,

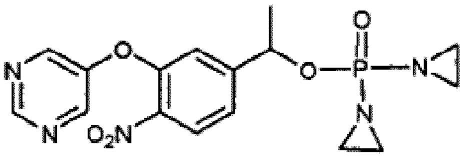,
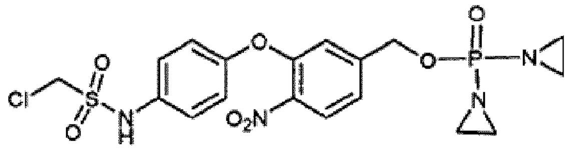,

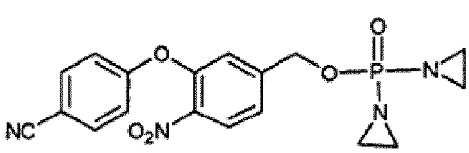,
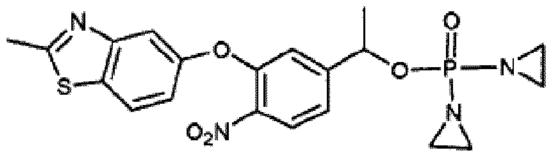,

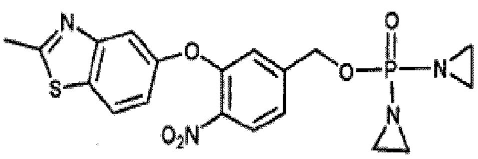,
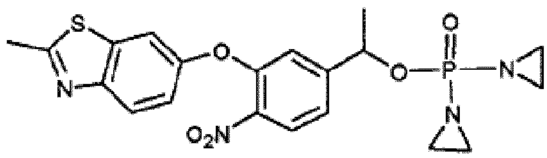,

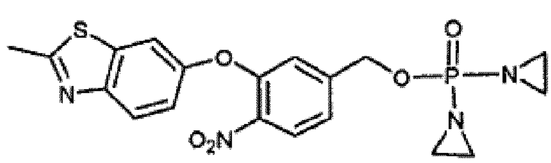,
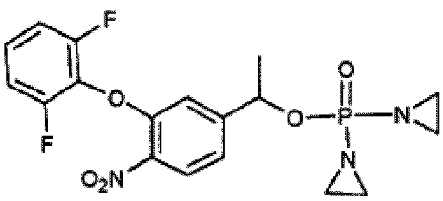,

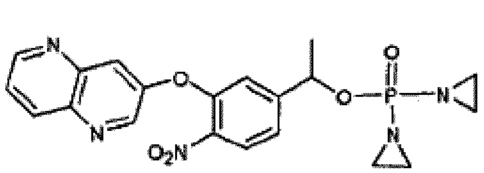,
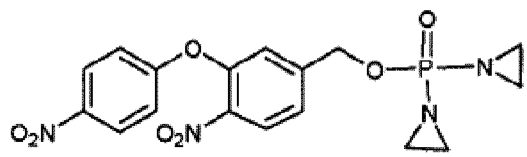,

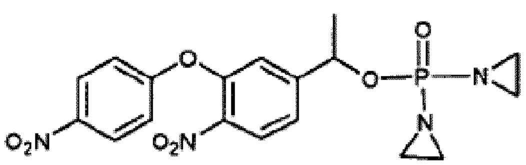,
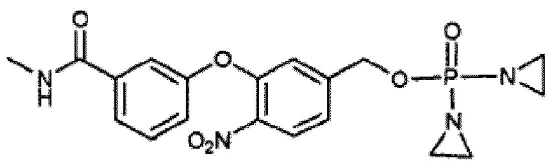,

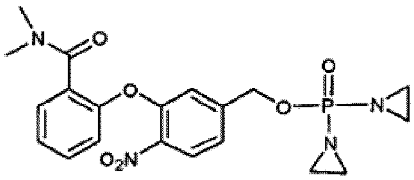,
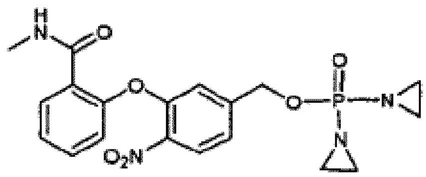,

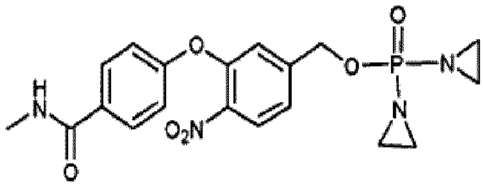,
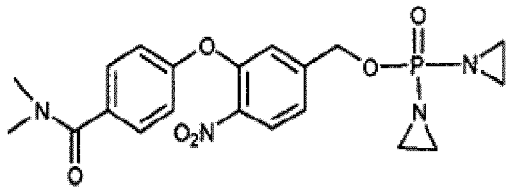,

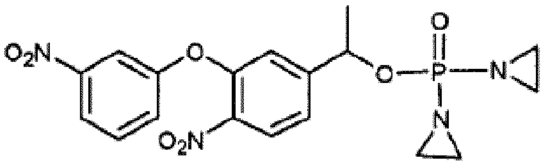,
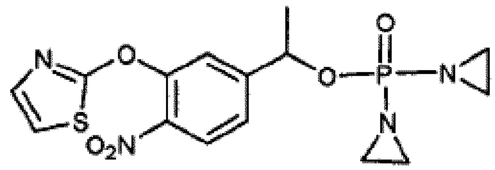,

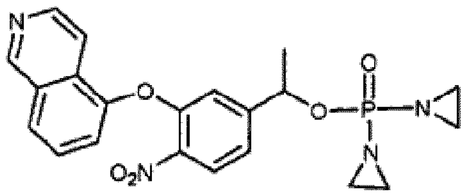,
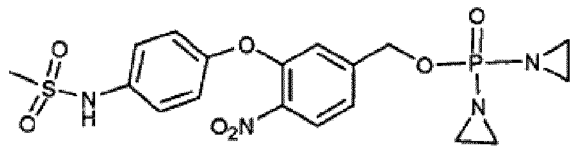,

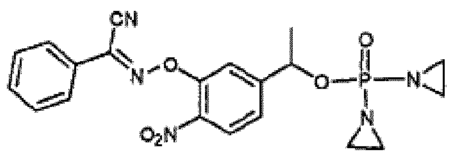,
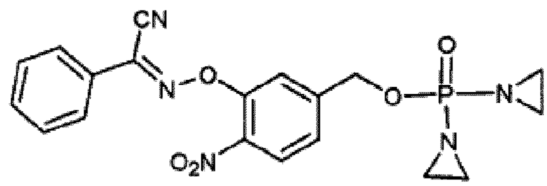,

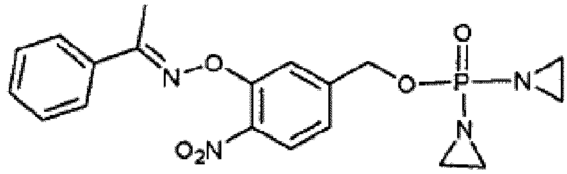,
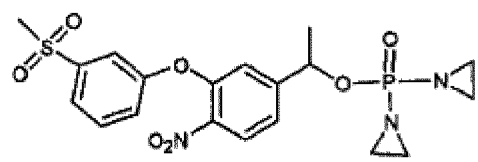,

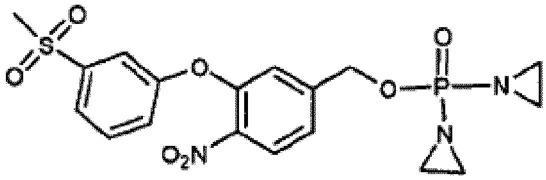,
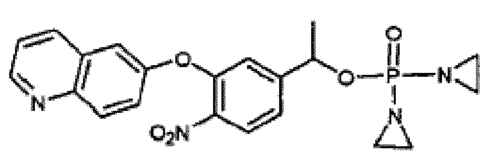,

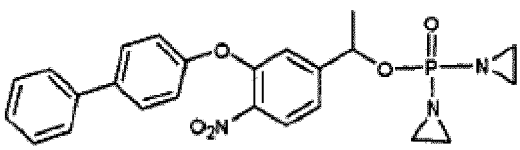,

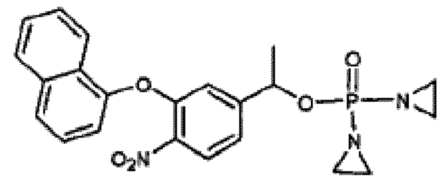,

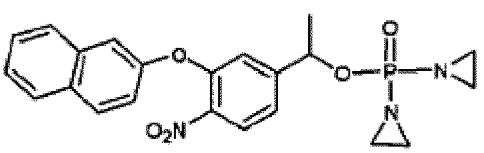,

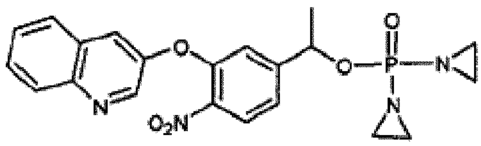,

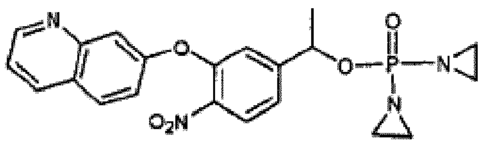,

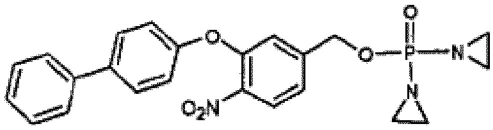,

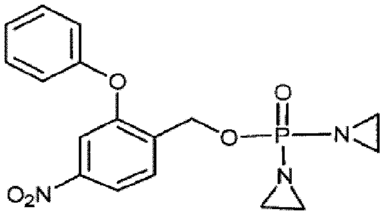,

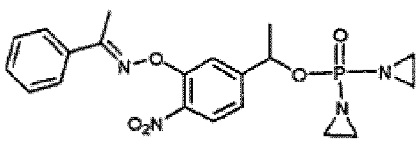,

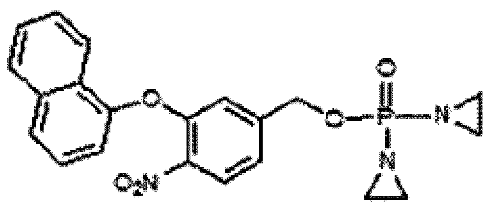,

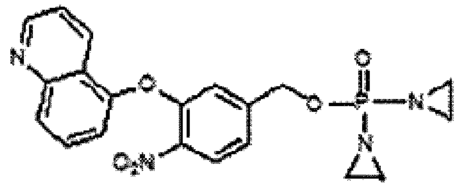,

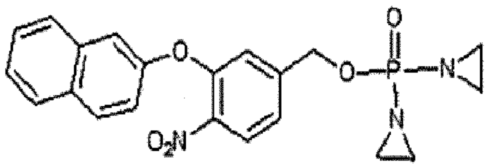,

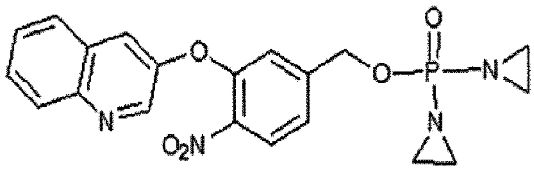,

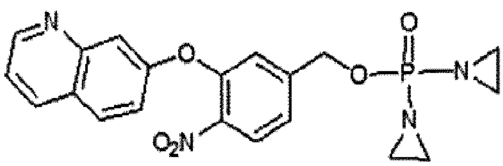,

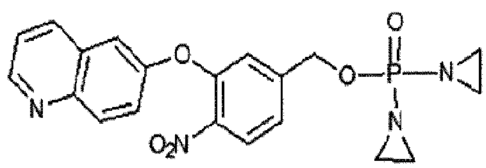,

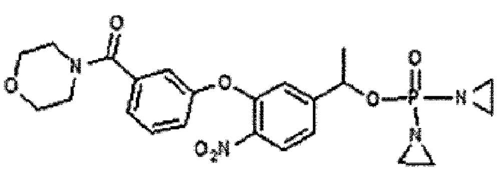, 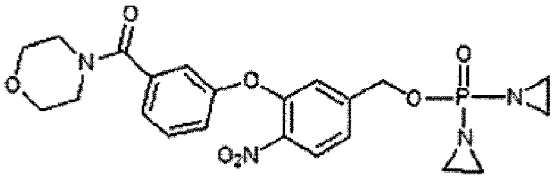,

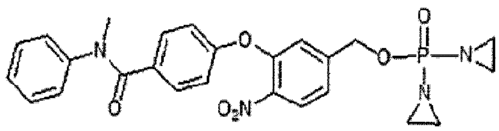, 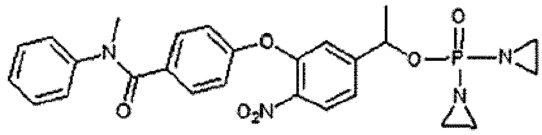,

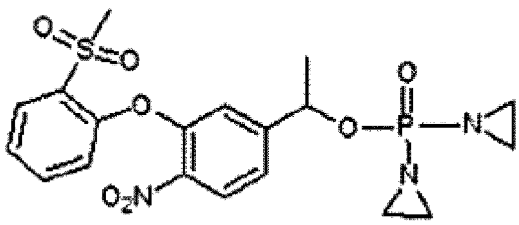, 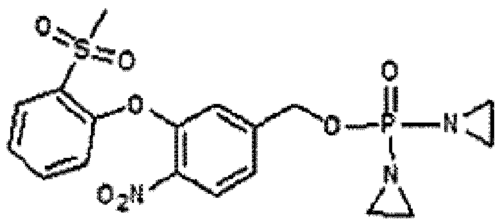,

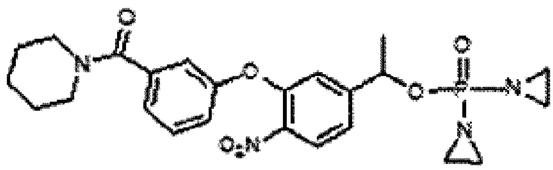, 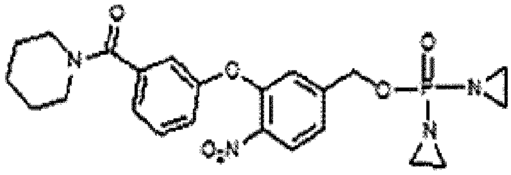,

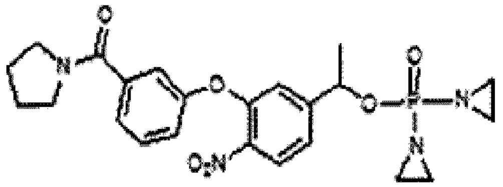, 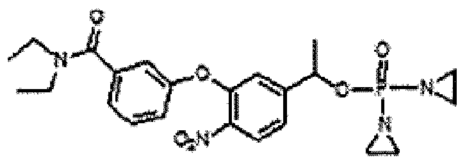,

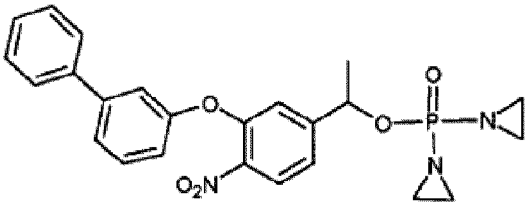, 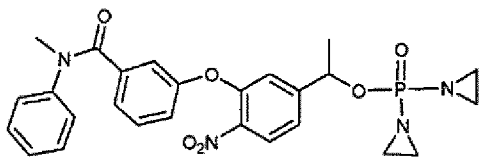,

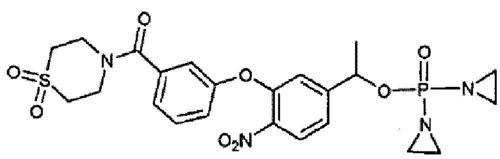, 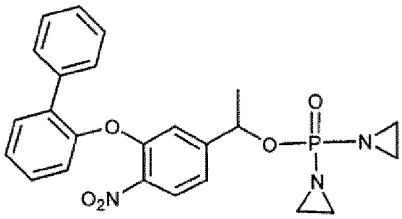,

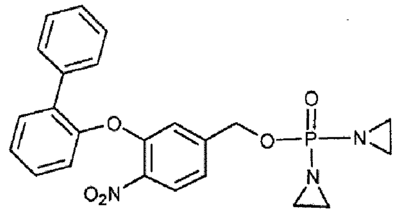, 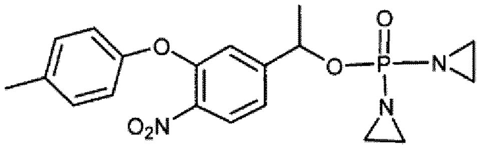,

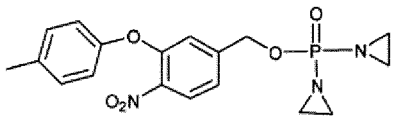, 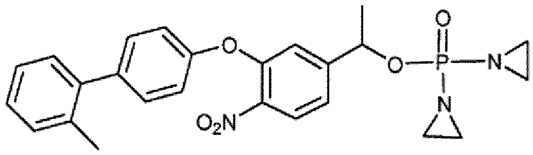,

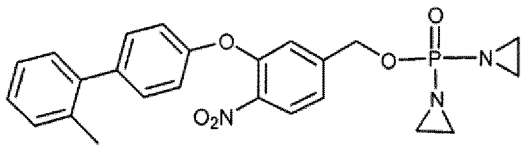, 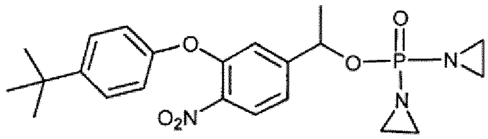,

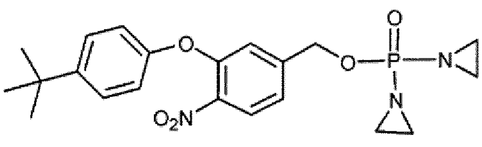, 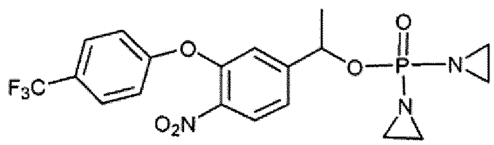,

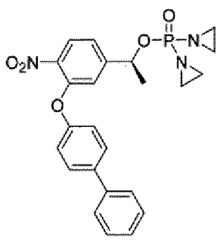, 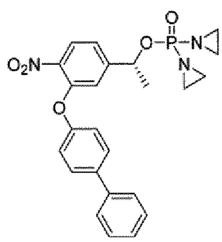,

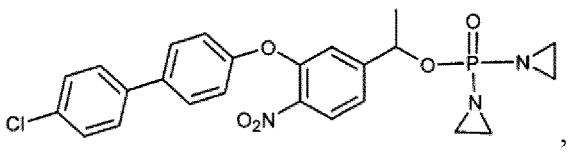, 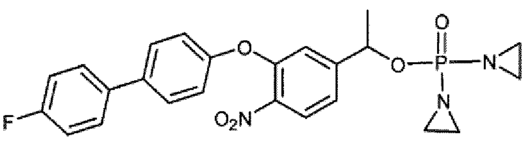,

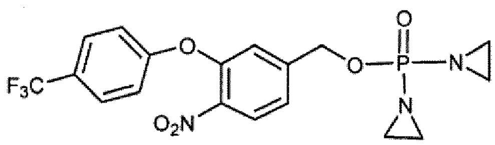 and 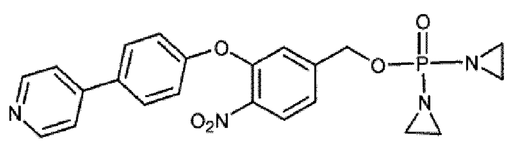.

According to Patent Application No. PCT/US2016/025665 (Publication No. WO2016161342), which corresponds to Chinese Patent Application No. 2016800446081 (Publication No. CN108290911A), the above compound of structural formula I further comprises:

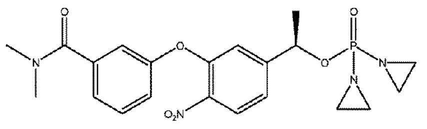 and 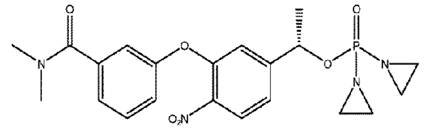 .

Further, in the above administration method, according to Patent Application No. PCT/US2016/062114 (Publication No. WO2017087428), which corresponds to Chinese Patent Application No. 2016800200132 (Publication No. CN108136214A), the AKR1C3 enzyme-activated anti-cancer prodrug contains a compound of structural formula II:

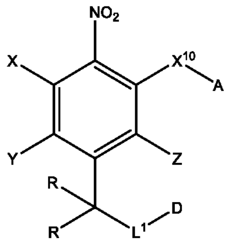

II

wherein

$X^{10}$ is O, S, SO or $SO_2$;

A is $C_6$-$C_{10}$ aryl or substituted aryl, 5-15 membered heteroaryl or substituted heteroaryl or -N=$CR^1R^2$; wherein $R^1$ and $R^2$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

X, Y and Z are each independently hydrogen, CN, halogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

each R is independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;

$R^{13}$ and $R^{14}$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl or ether, or $R^{13}$ and $R^{14}$ together with the nitrogen atom to which they are bonded form 5-7 membered heterocyclyl;

$L^1$ and D are as defined in the description, and the specific definitions are as follows:

$L^1$ is selected from:

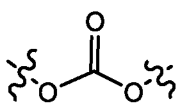, 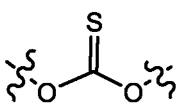, 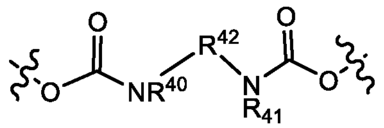, 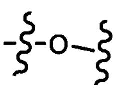,

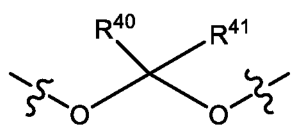 and 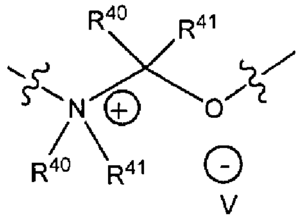 ;

wherein $R^{40}$ and $R^{41}$ are independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle or 5-15 membered heteroaryl;

$R^{42}$ is a $C_2$-$C_3$ alkylene or heteroalkylene optionally substituted with 1-3 $C_1$-$C_6$ alkyl; V(-) is any anion, preferably a pharmaceutically acceptable anion; and

D is a moiety that makes D-OH an anti-cancer drug, wherein OH is aliphatic hydroxyl or phenolic hydroxyl; in other words, D is a group after hydroxyl of the anti-cancer drug D-OH is removed;

or

$L^1$ is:

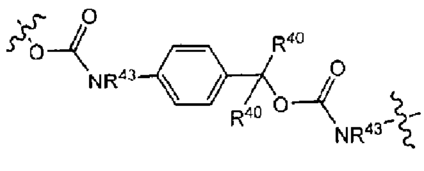
,
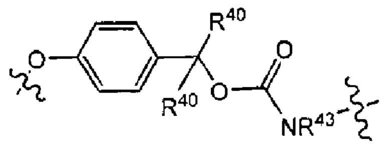
or
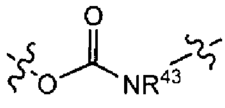
;

wherein $R^{40}$ is as defined above, $R^{43}$ is hydrogen or together with D forms heterocycle and the phenyl moiety is optionally substituted; and

D is a moiety that makes D-$NR^{43}$H an anti-cancer drug; in other words, D is a group after amino or amine of the anti-cancer drug D-$NR^{43}$H is removed;

or

$L^1$ is a bond, -O-C($R^{40}R^{41}$)-, -O-C($R^{40}R^{41}$)-$NR^{40}R^{41}$(+)-C($R^{40}R^{41}$)- or

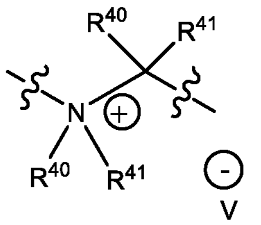
,

wherein $R^{40}$, $R^{41}$ and V are as defined above; and

D is an anti-cancer drug containing a primary or secondary amine, wherein the primary or secondary amine is bonded to $L^1$; and

alkyl, alkenyl, alkynyl, cycloalkyl, aryl, heterocycle, heteroaryl and the ether group are substituted or unsubstituted.

The substituent is a halogen atom, cyano or isocyano, thiocyano or isothiocyano, hydroxyl, mercapto, amino, oximido, a hydrazone group, OTs, OMS, $C_1$-$C_3$ alkyl or substituted alkyl, $C_1$-$C_3$ alkoxy or substituted alkoxy, $C_2$-$C_3$ alkenyl or substituted alkenyl, $C_2$-$C_3$ alkynyl or substituted alkynyl, $C_3$-$C_8$ cycloalkyl or substituted cycloalkyl, an aromatic ring, heterocycle, a heteroaromatic ring and a fused ring or a substituted aromatic ring, heterocycle, a heteroaromatic ring and a fused ring, wherein the substituted mode is monosubstituted or geminal disubstituted, and the substituent is a halogen atom, cyano or isocyano, thiocyano or isothiocyano, hydroxyl, mercapto, amino, oximido, a hydrazone group, OTs or OMs.

Further, in the above administration method, in the compound of structural formula II contained in the AKR1C3 enzyme-activated anti-cancer prodrug,

D-OH is selected from the following anti-cancer drugs containing an -OH group: gemcitabine, estramusting, pudnimnstine, chlorozotocin, ranimustine, mannomustine, mitobronitol, dibromodulcitol, aclacinomycins, anthramycin, bleomycin, carubicin, carzinophilin, chromomycin, actinomycin D, daunorubicin, mycophenolic acid, nogalamycin, olivomycin, peplomycin, plicamycin, puromycin, streptonigrin, streptozocin, tubercidin, ubenimex, zinostatin, zorubicin, denopterin, fludarabine, ancitabine, azacitidine, 6-azauridine, cytarabine, dideoxyuridine, doxifluridine, enocitabine, floxuridine, L-asparaginase, pulmozyme, aceglatone, elliptinium acetate, etoglucid, interferon-alpha, interferon-beta, interferon-gamma, interleukin-2, lentinan, mitoxantrone, mopidamol, pentostatin, pirarubicin, podophyllinic acid, sizofiran, paclitaxel, teniposide, tenuazonic acid, vinblastine and vincristine;

$NR^{43}H$ is selected from the following anti-cancer drugs: erlotinib, meturedepa, uredepa, imatinib, trimethylolomelamine, gefitinib, uracil mustard, carmustine, chlorozotocin, fotemustine, nimustine, ranimustine, dacarbazine, mannomustine, actinomycin, anthramycin, bleomycin, actinomycin C, carubicin, carzinophilin, actinomycin D, peplomycin, puromycin, streptozocin, ubenimex, zinostatin, denopterin, pteropterin, trimetrexate, 6-mercaptopurine, thiamiprine, thioguanine, 6-azauridine, carmofur, dideoxyuridine, doxifluridine, enocitabine, floxuridine, 5-fluorouracil, tegafur, L-asparaginase, pulmozyme, amsacrine, bisantrene, demecolcine, diaziquone, elliptinium acetate, flutamide, hydroxyurea, interferon-alpha, interferon-beta, interferon-gamma, interleukin-2, mitoxantrone, nitracrine, pentostatin, phenamet, 2-ethylhydrazide, procarbazine, razoxane, erlotonib, urethane, vinblastine and vincristine;

The anti-cancer drug containing a tertiary or secondary nitrogen atom is selected from: altretamine, triethylenemelamine, chlorambuci, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, pipobroman, actinomycin, anthramycin, carzinophilin, actinomycin D, nogalamycin, porfiromycin, puromycin, streptozocin, tubercidin, fludarabine, ancitabine, azacitidine, cytarabine, dideoxyuridine, enocitabine, floxuridine, L-asparaginase, pulmozyme, aldophosphamide glycoside, bestrabucil, diaziquone, interferon-alpha, interferon-beta, interferon-gamma, interleukin-2, mitoguazone, mopidamol, nitracrine, pentostatin, phenamet, razoxane, spirogermanium, tamoxifen, triaziquone, 2,2',2"-trichlorotriethylamine, vinblastine and vincristine.

Further, the AKR1C3 enzyme-activated anti-cancer prodrug (structural formula II) contains a compound having a structural formula selected from the group consisting of:

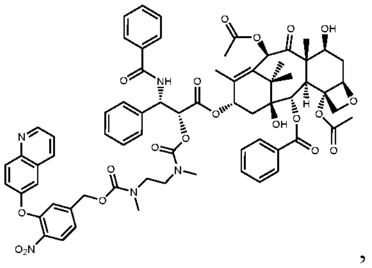
,
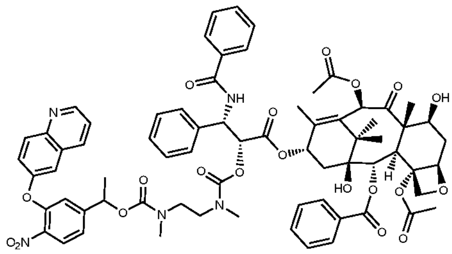
,

or

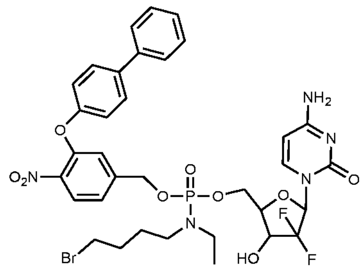
.

Scheme 3: An assembly for measuring the expression level of AKR1C3 enzyme

The present invention provides an assembly for measuring the expression level of AKR1C3 enzyme, comprising a reaction measurement component and a control comparison component.

The reaction measurement component is a component that contacts and reacts with the biological sample, and quantitatively or semi-quantitatively associate with the content of PGF2α and/or PGD2 and/or PGH2 in the biological

sample according to the signal of the reaction.

This type of component is similar to an ELISA test well plate.

The ELISA test well plate contains a reagent that can react with the target prostaglandin (F2α/D2/H2) and a special color reagent. Quantitation is performed by a photometer based on the depth of the reaction color.

The principle of the test well plate is similar to that of a commercially available ELISA kit. Quantitation is performed by the degree of a microplate reader or a photometer.

Obviously, in order to make the reaction measurement component react with the target prostaglandin accurately and without interference, the measurement component is also equipped with various corresponding auxiliary reagents: a reaction solution, a masking agent solution, a washing buffer solution, a reaction termination solution, and the like.

The control comparison component is a component that is used to compare the signal of the reaction to comparatively compare and obtain the expression level of AKR1C3 enzyme corresponding to the content of PGF2α and/or PGD2 and/or PGH2, the content change of PGF2α and/or PGD2 and/or PGH2 before and after administration of the interference drug, or the content change rate of PGF2α and/or PGD2 and/or PGH2 before and after administration of the interference drug in the biological sample.

This control comparison component can be a table of known corresponding relationship between concentration and absorbance: a user directly compares the different colors and the corresponding absorbances on the test well plate with the control comparison component and directly obtains the corresponding prostaglandin content level.

After the content level is obtained, the corresponding expression level of AKR1C3 enzyme can be directly obtained (Association Rule 1):

when the content of PGF2α is in the range of A1, the expression level of AKR1C3 enzyme in the biological sample is high;

when the content of PGF2α is in the range of B1, the expression level of AKR1C3 enzyme in the biological sample is medium;

when the content of PGF2α is in the range of C1, the expression level of AKR1C3 enzyme in the biological sample is low;

and/or

when the content of PGD2 and/or PGH2 is in the range of A2, the expression level of AKR1C3 enzyme in the biological sample is low;

when the content of PGD2 and/or PGH2 is in the range of B2, the expression level of AKR1C3 enzyme in the biological sample is medium;

when the content of PGD2 and/or PGH2 is in the range of C2, the expression level of AKR1C3 enzyme in the biological sample is high,

wherein the minimum value in the range of A1 is greater than or equal to the maximum value in the range of B1, and the minimum value in the range of B1 is greater than or equal to the maximum value in the range of C1,

the minimum value in the range of A2 is greater than or equal to the maximum value in the range of B2, and the minimum value in the range of B2 is greater than or equal to the maximum value in the range of C2.

Alternatively, the corresponding prostaglandin content grade is obtained after application of the interference drug, and the two are calculated to obtain the content grade difference (the content grade difference corresponds to the content difference within a range, which actually obtains a difference result of a semi-quantitative content) and the corresponding expression level of AKR1C3 enzyme can be obtained according to the following association (Association Rule two):

when the content change of PGF2α is in the range of a1, the expression level of AKR1C3 enzyme in the biological sample is high;

when the content change of PGF2α and/or PGD2 and/or PGH2 is in the range of b1, the expression level of AKR1C3 enzyme in the biological sample is medium;

when the content change of PGF2α and/or PGD2 and/or PGH2 is in the range of c1, the expression level of AKR1C3 enzyme in the biological sample is low;

and/or

when the content change of PGD2 and/or PGH2 is in the range of a2, the expression level of AKR1C3 enzyme in the biological sample is low;

when the content change of PGD2 and/or PGH2 is in the range of b2, the expression level of AKR1C3 enzyme in the biological sample is medium;

when the content change of PGD2 and/or PGH2 is in the range of c2, the expression level of AKR1C3 enzyme in the biological sample is high,

wherein the minimum value in the range of a1 is greater than or equal to the maximum value in the range of b1, and the minimum value in the range of b1 is greater than or equal to the maximum value in the range of c1,

the minimum value in the range of a2 is greater than or equal to the maximum value in the range of b2, and the minimum value in the range of b2 is greater than or equal to the maximum value in the range of c2.

This control comparison component can be a corresponding relationship table: a user uses a photometer or a microplate reader to test the fully reacted and treated test well plate and read it, and then obtains the corresponding prostaglandin content in the sample according to the reading versus a standard degree-content (concentration content, pg/ml concentration for human) curve. With the similar principle and operation as above, the more accurate content change rate can be used to determine the expression level of AKR1C3 enzyme (Association Rule III):

when the content change rate of PGF2α is in the range of α1, the expression level of AKR1C3 enzyme in the biological sample is high;

when the content change rate of PGF2α is in the range of β1, the expression level of AKR1C3 enzyme in the biological sample is medium;

when the content change rate of PGF2α is in the range of yl, the expression level of AKR1C3 enzyme in the biological sample is low,

and/or

when the content change rate of PGD2 and/or PGH2 is in the range of α2, the expression level of AKR1C3 enzyme in the biological sample is low;

when the content change rate of PGD2 and/or PGH2 is in the range of β2, the expression level of AKR1C3 enzyme in the biological sample is medium;

when the content change rate of PGD2 and/or PGH2 is in the range of γ2, the expression level of AKR1C3 enzyme in the biological sample is high,

wherein the minimum value in the range of α1 is greater than or equal to the maximum value in the range of β1, and the minimum value in the range of β1 is greater than or equal to the maximum value in the range of γ1,

the minimum value in the range of α2 is greater than or equal to the maximum value in the range of β2, and the minimum value in the range of β2 is greater than or equal to the maximum value in the range of γ2.

It is known to those skilled in the art that although the above description is only based on the content level, it is also feasible to use the photometer or the microplate reader to directly read the test well plate to obtain the corresponding prostaglandin content, and then use Association Rule 1 or Rule 2 to determine, or it is also feasible to use Rule 2 or Rule 3 according to the content change value and content change rate to determine. The above description is not limited to a certain corresponding limit.

Obviously, the above method is only one of the methods for measuring prostaglandins. In fact, reaction measurement components of different formulations corresponding to PGF2α, PGD2, and PGH2 can be used for quantitatively or semi-quantitatively measuring different prostaglandin contents. Then different corresponding relationship tables are correspondingly established to respectively measure the contents of different prostaglandins, the content change and the content change rate before and after the application of the interference drug, so that the test component can be applied to a wider population, and the test result is more accurate and credible.

Scheme 4: Administration device

The present invention provides an administration device, comprising:

the assembly for measuring the expression level of AKR1C3 enzyme as described above;

An adminstration assembly, which contains an AKR1C3 enzyme-activated anti-cancer prodrugs.

Obviously, the administration device is equipped with a further administration assembly on the basis of the above-mentioned test assembly: including an administration tool and a drug.

For example, the adminstration tool can be a injector, and the corresponding drug is a powder for injection (lyophilized or aseptic packing) and a diluted solution for injection; or the injector and the drug are designed as an integrated structure, which is directly injected for administration without further operations of dilution or dissolution.

The adminstration tool can be a hypospray, and the corresponding drug is a powder for injection (lyophilized or aseptic packing), i. e. a so-called "needle-free injection system", which relies on propulsion of compressed gas or explosion to inject the powder through the skin into the blood vessels.

## Brief Description Of The Drawings

Figure 1 is a schematic diagram of metabolism principle of DNA alkylated anti-cancer prodrugs targeting Aldo-keto reductase 1C3 (AKR1C3).
Figure 2 is a schematic diagram showing the effect of AKR1C3 on metabolism of prostaglandin.
Figure 3 is a schematic diagram showing the effect of AKR1C3 inhibitors on metabolism of prostaglandin.
Figure 4 is a schematic diagram showing the effect of AKR1C3 agonists on metabolism of prostaglandin.

## Detailed Description of the Invention

The present invention will be described below with reference to specific examples. Those skilled in the art could understand that these examples are only used for describing the invention and do not in any way limit its scope.

The experimental methods in the following examples are all conventional methods unless otherwise specified. The raw materials of the drugs, the reagents and the like used in the following examples are all commercially available products unless otherwise specified.

The following definitions are provided to assist the reader. Unless otherwise defined, all terms of art, notations, and other scientific or medical terms or terminology used herein are intended to have the meanings commonly understood by those of skill in the chemical and medical arts. In some cases, terms with commonly understood meanings are defined herein for clarity and/or for ready reference, and the inclusion of such definitions herein should not be construed as representing a substantial difference from the definitions of the terms as generally understood in the art.

All numerical designations, e.g., pH, temperature, time, concentration, and weight, including ranges of each thereof, are approximations that typically may be varied (+) or (-) by increments of 0.1, 1.0, or 10.0, as appropriate. All numerical designations may be understood as preceded by the term "about". Reagents described herein are exemplary and equivalents of such may be known in the art.

"$C_x$-$C_y$" or "$C_{x-y}$" before a group refers to a range of the number of carbon atoms that are present in that group. For example, $C_1$-$C_6$ alkyl refers to an alkyl group having at least 1 and up to 6 carbon atoms.

"Alkoxy" refers to -O-Alkyl.

"Amino" refers to $NR^pR^q$ wherein $R^p$ and $R^q$ independently are hydrogen or $C_1$-$C_6$ alkyl, or $R^p$ and $R^q$ together with the nitrogen atom to which they are bonded form a 4-15 membered heterocycle.

"Aryl" refers to an aromatic group having carbon atoms and containing no ring heteroatoms and having a single ring (e.g., phenyl) or multiple condensed (fused) rings (e.g., naphthyl or anthryl). For multiple ring systems, including fused, bridged, and spiro ring systems having aromatic and non-aromatic rings that have no ring heteroatoms, the term "aryl" or "Ar" applies when the point of attachment is at an aromatic carbon atom (e.g., 5,6,7,8 tetrahydronaphthalene-2-yl is an aryl group, as its point of attachment is at the 2-position of the aromatic phenyl ring).

According to specific embodiments of the present application, $C_6$-$C_{10}$ aryl can be phenyl, naphthyl and various substituted phenyl or naphthyl.

"Heteroaryl" (Heterocyclic aryl) refers to an aromatic group of from 1 to 14 carbon atoms and 1 to 6 heteroatoms selected from the group consisting of oxygen, nitrogen, and sulfur and includes single ring (e.g. imidazolyl-2-yl and imidazol-5-yl) and multiple ring systems (e.g. imidazopyridyl, benzotriazolyl, benzimidazol-2-yl and benzimidazol-6-yl). For multiple ring systems, including fused, bridged, and spiro ring systems having aromatic and non-aromatic rings, the term "heteroaryl" applies if there is at least one ring heteroatom, and the point of attachment is at an atom of an aromatic ring (e.g. 1,2,3,4-tetrahydroquinolin-6-yl and 5,6,7,8-tetrahydroquinolin-3-yl). In some embodiments, the nitrogen and/or the sulfur ring atom(s) of the heteroaryl group are optionally oxidized to provide for the N-oxide ($N\rightarrow O$), sulfinyl, or sulfonyl moieties. The term heteroaryl or 5-15 membered heteroaryl includes, but is not limited to, acridinyl, azocinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, benzothienyl, benzimidazolinyl, carbazolyl, NH-carbazolyl, carbolinyl, chromanyl, chromenyl, cinnolinyl, dithiazinyl, furanyl, furazanyl, imidazolidinyl, imidazolinyl, imidazopyridyl, imidazolyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolyl, isoxazolyl, naphthyridinyl, octahydroisoquinolinyl, oxadiazolyl, oxazolidinyl, oxazolyl, pyrimidinyl, phenanthridinyl, phenanthrolinyl, phenazinyl, phenothiazinyl, phenoxathiinyl, phenoxazinyl, phthalazinyl, piperazinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazole, pyridinyl, pyridyl, pyrimidinyl, pyrrolyl, quinazolinyl, quinolinyl, quinoxalinyl, quinuclidinyl, tetrahydroisoquinolinyl, tetrahydroquinolinyl, tetrazolyl, thiadiazinyl, thiadiazolyl, thianthrenyl, thiazolyl, thienyl, thienothiazolyl, thienooxazolyl, thienoimidazolyl, thiophenyl, triazinyl and xanthenyl.

"Alkyl" refers to a monovalent saturated aliphatic hydrocarbyl group having carbon atom(s) and, in some embodiments, from 1 to 6 carbon atoms. "$C_{x-y}$ alkyl" refers to an alkyl group having from x to y carbon atoms. This term includes, by way of example, linear and branched hydrocarbyl groups such as methyl ($CH_3$-), ethyl ($CH_3CH_2$-), n-propyl ($CH_3CH_2CH_2$-), isopropyl ($(CH_3)_2CH$-), n-butyl ($CH_3CH_2CH_2CH_2$-), isobutyl ($(CH_3)_2CHCH_2$-), sec-butyl ($(CH_3)(CH_3CH_2)CH$-), tert-butyl ($(CH3)_3C$-), n-pentyl ($CH_3CH_2CH_2CH_2CH_2$-), and neopentyl ($(CH_3)_3CCH_2$-).

"Cycloalkyl" refers to a saturated or partially saturated cyclic group with more than 3 carbon atoms and no ring heteroatoms and having a single ring or multiple rings including fused, bridged, and spiro ring systems. For multiple ring systems having aromatic and non-aromatic rings that have no ring heteroatoms, the term "cycloalkyl" applies when the point of attachment is at a non-aromatic carbon atom (e.g. 5,6,7,8-tetrahydronaphthalene-5-yl). The term "cycloalkyl" or "$C_3$-$C_8$ cycloalkyl" includes cycloalkenyl groups. Examples of cycloalkyl groups or $C_3$-$C_8$ cycloalkyl groups include, for instance, adamantyl, cyclopropyl, cyclobutyl, cyclopentyl, cyclooctyl, and cyclohexenyl.

"Heterocyclic" or "heterocycle" or "heterocycloalkyl" or "heterocyclyl" refers to a saturated or partially saturated cyclic group having carbon atom(s) and from 1 to 6 heteroatoms selected from the group consisting of nitrogen, sulfur, or oxygen and includes single ring and multiple ring systems including fused, bridged, and spiro ring systems. For multiple ring systems having aromatic and/or non-aromatic rings, term "heterocyclic", or "heterocycle", or "heterocycloalkyl", or "heterocyclyl" applies when there is at least one ring heteroatom, and the point of attachment is at an atom of a non-aromatic ring (e.g. 1,2,3,4-tetrahydroquinoline-3-yl, 5,6,7,8-tetrahydroquinoline-6-yl, and decahydroquinolin-6-yl). In some embodiments, the heterocyclic groups herein are 3-15 membered, 4-14 membered, 5-13 membered, 7-12 membered, or 5-7 membered heterocycles. In some other embodiments, the heterocycles contain 4 heteroatoms. In some other embodiments, the heterocycles contain 3 heteroatoms. In another embodiment, the heterocycles contain up to 2 heteroatoms. In some embodiments, the nitrogen and/or sulfur atom(s) of the heterocyclic group are optionally oxidized to provide for the N-oxide, sulfinyl, or sulfonyl moieties. Heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (e.g., $C_3$-$_{10}$) refers to the total number of carbon atoms in the portion of the heterocyclyl group exclusive of the number of heteroatoms. A divalent heterocyclic group will have the appropriately adjusted hydrogen content.

"Ether" refers to a $C_1$-$C_6$ alkyl group substituted with 1-3 $C_1$-$C_6$ alkoxy groups, wherein alkoxy refers to -O-alkyl.

"Halo" or "Halogen" refers to one or more of fluoro, chloro, bromo, and iodo.

"Alkenyl" refers to a linear or branched hydrocarbyl group having carbon atoms and in some embodiments from 2 to 6 carbon atoms or 2 to 4 carbon atoms and having at least 1 site of vinyl unsaturation (>C=<). For example, $C_{x-y}$ alkenyl refers to an alkenyl group having from x to y carbon atoms and is meant to include, for example, ethenyl, propenyl, 1,3-butadienyl, and the like.

"Alkynyl" refers to a linear monovalent hydrocarbon group or a branched monovalent hydrocarbon group having more than 2 carbon atoms and in some embodiments from 2 to 6 carbon atoms or 2 to 4 carbon atoms and containing at least one triple bond. The term "alkynyl" is also meant to include those hydrocarbyl groups having one triple bond and one double bond. For example, $C_{2-6}$ alkynyl includes ethynyl, propynyl, and the like.

"Phosphoramidate alkylating agent" refers to an alkylating agent comprising one or more $Z^5$-$X^5$-$Y^5$ moieties

bonded to an -O-P(Z1) moiety, where $Z^5$ is a heteroatom such as nitrogen, sulfur or oxygen, $X^5$ is optionally substituted ethylene, $Y^5$ is halo or another leaving group, or $Z^5$-$X^5$-$Y^5$ together form an aziridinyl ($NCH_2CH_2$) moiety, and $Z^1$ is defined as above. Such an alkylating agent can react with DNA or another nucleic acid or protein. In some instances, an alkylating agent can be cross-linked with DNA.

The group may be substituted with one or more substituents, e.g., 1, 2, 3, 4 or 5 substituents. Preferably, the substituents are selected from the group consisting of oxo, halo, -CN, $NO_2$, $-N_2+$, $-CO_2R^{100}$, $-OR^{100}$, $-SR^{100}$, $-SOR^{100}$, $-SO_2R^{100}$, $-NR^{100}SO_2R^{100}$, $-NR^{101}R^{102}$, $-CONR^{101}R^{102}$, $-SO_2NR^{101}R^{102}$, $C_1$-$C_6$ alkyl, $C_1$-$C_6$ alkoxy, $-CR^{100} = C (R^{100})_2$, $-CCR^{100}$, $C_3$-$C_{10}$ cycloalkyl, $C_3$-$C_{10}$ heterocyclyl, C6-Ci2 aryl and $C_2$-$C_{12}$ heteroaryl, or a divalent substituent such as $-O-(CH_2)-O-$, $-O-(CH_2)_2-O-$, and, 1-4 substituted methyl groups , wherein each $R^{100}$, $R^{101}$, and $R^{102}$ independently is hydrogen or $C_1$-$C_8$ alkyl; $C_3$-$C_{12}$ cycloalkyl; $C_3$-$C_{10}$ heterocyclyl; $C_6$-$C_{12}$ aryl; or $C_2$-$C_{12}$ heteroaryl; or $R^{100}$ and $R^{102}$ together with the nitrogen atom to which they are attached form a 5-7 membered heterocycle; wherein each alkyl, cycloalkyl, heterocyclyl, aryl, or heteroaryl is optionally substituted with 1-3 halo, 1-3 $C_1$-$C_6$ alkyl, 1-3 $C_1$-$C_6$ haloalkyl or 1-3 $C_1$-$C_6$ alkoxy groups. Preferably, the substituents are selected from the group consisting of chloro, fluoro, $-OCH_3$, methyl, ethyl, iso-propyl, cyclopropyl, $-CO_2H$ and salts and $C_1$-$C_6$ alkyl esters thereof, $CONMe_2$, CONHMe, $CONH_2$, $-SO_2Me$, $-SO_2NH_2$, $-SO_2NMe_2$, $-SO_2NHMe$, $-NHSO_2Me$, $-NHSO_2CF_3$, $-NHSO_2CH_2Cl$, $-NH_2$, $-OCF_3$, $-CF_3$ and $-OCHF_2$.

"Alkylene" refers to a divalent saturated aliphatic hydrocarbyl group having carbon atom(s) and, in some embodiments, from 1 to 6 carbon atoms, and an alkyl group further losing one H atom. "$C_{u-v}$ alkylene" refers to alkylene groups having from u to v carbon atoms. The alkylene group includes branched and straight chain hydrocarbyl groups. For example, "$C_{1-6}$ alkylene" includes methylene, ethylene, propylene, 2-methylpropylene, pentylene, and the like.

"Heteroalkylene" refers to an alkylene wherein a chain carbon atom is replaced with a heteroatom such as O, S, N, or P, or a heteroatom containing a substituent.

The "drug" regarding D herein includes without limitation, gemcitibine, erlotinib, meturedepa, uredepa, altretamine, imatinib, triethylenemelamine, trimethylmelamine, chlorambucil, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, mannomustine, mitobronitol, mitolactol, pipobroman, aclacinomycins, actinomycin, anthramycin, azaserine, bleomycin, actinomycin C, carubicin, carzinophilin, chromomycin, actinomycin D, daunorubicin, daunomycin, 6-diazo-5-oxo-1-norleucine, mycophenolic acid, nogalamycin, olivomycin, peplomycin, plicamycin, porfiromycin, puromycin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, 5-fluorouracil, tegafur, L-asparaginase, pulmozyme, aceglatone, aldophosphamide glycoside, aminolevulinic acid, amsacrine, bestrabucil, bisantrene, defofamide, demecolcine, diaziquone, elfornithine, elliptinium acetate, etoglucid, flutamide, hydroxyurea, interferon-alpha, interferon-beta, interferon-gamma, interleukin-2, lentinan, mitoguazone, mitoxantrone, mopidamol, nitracrine, pentostatin, phenamet, pirarubicin, podophyllinic acid, 2-ethylhydrazide, procarbazine, razoxane, sizofiran, spirogermanium, paclitaxel, tamoxifen, erlotonib, teniposide, tenuazonic acid, triaziquone, 2,2',2"-trichlorotriethylamine, urethane, vinblastine, and vincristine.

"Administering" or "administration of' a drug to a patient (and grammatical equivalents of this phrase) refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is to administer the drug to the patient.

"Cancer" refers to leukemias, lymphomas, carcinomas, and other malignant tumors, including solid tumors, of potentially unlimited growth that can expand locally by invasion and spread throughout the body by metastasis. Examples of cancers include, but are not limited to, cancer of the adrenal gland, bone, brain, breast, bronchi, colon and/or rectum, gallbladder, head and neck, kidney, larynx, liver, lung, neural tissue, pancreas, prostate, parathyroid, skin, stomach, and thyroid. Certain other examples of cancers include, acute and chronic lymphocytic and granulocytic tumors, adenocarcinoma, adenoma, basal cell carcinoma, poor cervical intraepithelial differentiation and in situ carcinoma, Ewing's sarcoma, epidermoid carcinomas, giant cell tumor, glioblastoma multiforma, hairy-cell tumor, intestinal ganglioneuroma, hyperplastic corneal nerve tumor, islet cell carcinoma, Kaposi's sarcoma, leiomyoma, leukemias, lymphoma, malignant carcinoid, malignant melanoma, malignant hypercalcemia, marfanoid habitus tumor, medullary epithelial carcinoma, metastatic skin carcinoma, mucosal neuroma, myeloma, mycosis fungoides, neuroblastoma, osteo sarcoma, osteogenic and other sarcoma, ovarian tumor, pheochromocytoma, polycythermia vera, primary brain tumor, small-cell lung tumor, squamous cell carcinoma of both ulcerating and papillary type, hyperplasia, seminoma, soft tissue sarcoma, retinoblastoma, rhabdomyosarcoma, renal cell tumor, topical skin lesion, reticulum cell sarcoma, and Wilm's tumor.

"Patient" and "subject" are used interchangeably to refer to a mammal in need of treatment for cancer. Generally, the patient is a human. Generally, the patient is a human diagnosed with cancer. In certain embodiments, a "patient" or "subject" may refer to a non-human mammal used in screening, characterizing, and evaluating drugs and therapies, such as, a non-human primate, a dog, cat, rabbit, pig, mouse or a rat.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is made such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

The patient in the present application refers to a patient suffering from a disease or disorder, complication associated with the AKR1C3 enzyme and its corresponding gene, or further defined as a cancer or tumor corresponding to the cytotoxic prodrug activated by the AKR1C3 enzyme or disorder or cell proliferative disease caused by the cancer or tumor.

"Solid tumor" refers to solid tumors including, but not limited to, metastatic tumors in bone, brain, liver, lung, lymph node, pancreas, prostate, skin and soft tissue (sarcoma).

"Therapeutically effective amount" of a drug refers to an amount of a drug that, when administered to a patient with cancer, will have the intended therapeutic effect, e.g., alleviation, amelioration, palliation or elimination of one or more manifestations of cancer in the patient. A therapeutic effect does not necessarily occur by administration of one dose, and may occur only after administration of a series of doses. Thus, a therapeutically effective amount may be administered in one or more administrations.

"Treatment of" a condition or patient refers to taking steps to obtain beneficial or desired results, including clinical results. For purposes of this invention, beneficial or desired clinical results include, but are not limited to, alleviation or improvement of one or more symptoms of cancer; diminishment of extent of disease; delay or slowing of disease progression; alleviation, palliation, or stabilization of the disease state; or other beneficial results. Treatment of cancer may, in some cases, result in partial response or stable disease.

"Tumor cells" refers to tumor cells of any appropriate species, e.g., mammalian such as murine, canine, feline, equine or human.

The above description of embodiments of the present invention does not limit the present invention. Those skilled in the art can make various modifications and changes according to the present invention, and any modification and change within the spirit of the present invention shall be covered in the scope of the claims appended to the present invention.

Since the present invention is based on the following three invention applications: Application No. PCT/US2016/021581, publication No. WO2016/145092 corresponding to Chinese application No. 2016800150788, publication No. CN107530556A;

Application No. PCT/US2016/025665, publication No. WO2016/061342 corresponding to Chinese application No. 2016800200132, publication No. CN108136214A; and
Application No. PCT/US2016/062114, publication No. WO2017/087428 corresponding to Chinese application No. 2016800446081 , publication No. CN108290911A, the above-mentioned three applications are incorporated into the text of the present application for this purpose.

Further, the AKR1C3 inhibitors mentioned in the present invention are for this purpose also incorporated into the present application by reference to the following applications:

| Publication No. | Patent application title | Application No. |
|---|---|---|
| US20130116277A1 | Aldo-keto reductase subfamily 1c3 (akrlc3) inhibitors | US13607633 |
| US20180305305A1 | 2-beta-naphthyl-acetic acid analogs as akrlc3 inhibitors and methods of using same | US15769565 |
| US20160159731A1 | Bifunctional akrlc3 inhibitors/androgen receptor modulators and methods of use thereof | US14993742 |
| US20140107085A1 | Bifunctional akrlc3 inhibitors/androgen receptor modulators and methods of use thereof | US14050937 |
| US20170260226A1 | 3-nitrogen or sulphur substituted oestra-1,3,5(10),16-tetraene akrlc3 inhibitors | US15510348 |

(continued)

| Publication No. | Patent application title | Application No. |
|---|---|---|
| US20160303082A1 | Indomethacin analogs for the treatment of castrate-resistant prostate cancer | US15132937 |
| US20180271835A1 | Indomethacin analogs for the treatment of castrate-resistant prostate cancer | US15899171 |
| US20140371261A1 | Indomethacin analogs for the treatment of castrate-resistant prostate cancer | US14352421 |
| US20140249119A1 | Estra-1,3,5(10),16-tetraene-3-carboxamide derivatives, processes for their preparation, pharmaceutical preparations comprising them and their use for preparing medicaments | US14348645 |
| US20150210734A1 | 3 -sub stituted estra-1,3,5(10), 16-tetraene derivatives, methods for the production thereof, | US14414386 |
| | pharmaceutical preparations containing same, and use thereof for the production of medicaments | |
| US20160024142A1 | Estra-1,3,5(10),16-tetraene-3-carboxamides for inhibition of 17.beta.-hydroxysteroid dehydrogenase (akr1 c3) | US14770444 |
| US20090275608A1 | Methods of diagnosing and treating parp-mediated diseases | US12322551 |
| US20170342082A1 | [8-(phenylsulfonyl)-3,8-diazabicyclo[3.2.1]oct-3-yl ](1h-1,2,3-triazol-4-yl)methanones | US15596383 |
| US20180319807A2 | [8-(phenylsulfonyl)-3,8-diazabicyclo[3.2.1]oct-3-yl ](1h-1,2,3-triazol-4-yl)methanones | US15596383 |
| CN201811133143.4 | A dihydropyranopyrazole compound, preparation method and application thereof | CN109305972A |

With regard to the AKR1C3 inhibitors mentioned in the present invention, it has been reported in the following literatures (Higaki, Y, Usami, et al. Selective and potent inhibitors of human 20a-hydroxysteroid dehydrogenase (AKR1C1) that metabolizes neurosteroids derived from progesterone[J]. CHEMICOBIOLOGICAL INTERACTIONS, 2003, 503-513; Bydal P , Luu-The V , Labrie F , et al. Steroidal lactones as inhibitors of 17beta-hydroxysteroid dehydrogenase type 5: chemical synthesis, enzyme inhibitory activity, and assessment of estrogenic and androgenic activities.[J]. European Journal of Medicinal Chemistry, 2009, 44(2):632-644; Skarydova L , Wsol V , Zivna L , et al. AKR1C3 as a potential target for the inhibitory effect of dietary flavonoids[J]. Chemico-biological interactions, 2010, 178:138-144; Byrns M C , Steckelbroeck S , Penning T M . An indomethacin analogue, N-(4-chlorobenzoyl)-melatonin, is a selective inhibitor of aldo-keto reductase 1C3 (type 2 3alpha-HSD, type 5 17beta-HSD, and prostaglandin F synthase), a potential target for the treatment of hormone dependent and hormone independent malignancies.[J]. Biochemical Pharmacology, 2008, 75(2):484-493; Bauman, D. R . Development of nonsteroidal anti-inflammatory drug analogs and steroid carboxylates selective for human aldo-keto reductase isoforms: potential antineoplastic agents that work independently of cyclooxygenase isozymes.[J]. Molecular Pharmacology, 2005, 67(1):60-68 ; Penning, T, M, et al. Inhibition of a major NAD(P)-linked oxidoreductase from rat liver cytosol by steroidal and nonsteroidal anti-inflammatory agents and by prostaglandins.[J]. Proceedings of the National Academy of Sciences, 1983, 8:4504-4508; Davies N J , Hayden R E , Simpson P J , et al. AKR1C isoforms represent a novel cellular target for jasmonates alongside their mitochondrial-mediated effects.[J]. Cancer Research, 2009, 69(11):4769-75; Brozic P , Golob B , Gomboc N , et al. Cinnamic acids as new inhibitors of 17beta-hydroxysteroid dehydrogenase type 5 (AKR1C3).[J]. Molecular & Cellular Endocrinology, 2006, 248(1-2):233-235; Yining Zhao, Xuehua Zheng, Hong Zhang, et al. Invitro inhibition of AKRICs by sulphonylureas and the structural basis.[J]. Chemico-Biological Interactions, 2015, 240:310-315.) that some commercially available drugs have an inhibitory effect on AKR1C3, and these drugs are also inhibitors of the AKR1C3 enzyme:
Glycyrrhetinic acid, Glycyrrhizinate and salt or glycoside thereof,Ursodeoxycholic acid, medroxyprogesterone acetate (MPA), Estradiol, Hexestrol, Bethamethasone, cortisone, Prednisone, methylprednisolone, triamcinolone, Hydrocortisone, Dexamethasone, Spironolactone, Brotizolam, Estazolam, Flunitrazepam, Flurazepam, meclonazepan, Lormetazepam, Midazolam, nimetazepam, Nitrazepam, Temazepam, triazolam, alprazolam, Bromazepam, Chlordiazepoxide, clobazam, clonazepam, Delorazepam, diazepam, fludiazepam, Halazepam, Lorazepam, Medazepam,

nordazepam, oxazepam, Prazepam, Cloxazolam, Phenolphthalein, Ipriflavone, Flavoxate Hydrochloride, Purified Micronised Flavonoid Fraction, Seabuckthorn flavone, silibinin, efloxate, Quercetin, Luteolin, Aspirin, Sodium salicylate, Paracetamol, Naproxen, Nabumetone, Diclofenac, Ibuprofen, rofecoxib, Celecoxib, Aceclofenac, Diflunisal, Etodolac, Fenoprofen, Flurbiprofen, ketoprofen, suprofen, Tiaprofenic Acid, Ketorolac, Zomepirac, Mefenamic acid, Flufenamic acid, Meclofenamic Acid, Meloxicam, Oxaprozin, Piroxicam, Tenoxicam, Lornoxicam, Sasapyrine, Sulindac, Tolmetin, Phenacetin, Loxoprofen Sodium, Aminopyrine, Metamizole Sodium, Sudoxicam, Phenylbutazone, Oxyphenbutazone, Chlorpropamide, Tolbutamide, Gliclazide, Glibenclamide, Gliquidone, Glipizide, and Glimepiride.

The following are specific tests and examples of the present invention.

The following tests will disclose experimental data of the applicant for proving relevant conclusions and relevant experimental facts of the present application, and the applicant hereby declares that the rights of the following experimental data belong to the applicant.

The compound

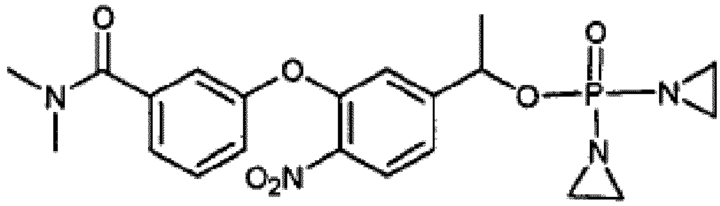

(i.e., the racemic isomer of compound AST-3424, the S-configuration of which is AST-3424

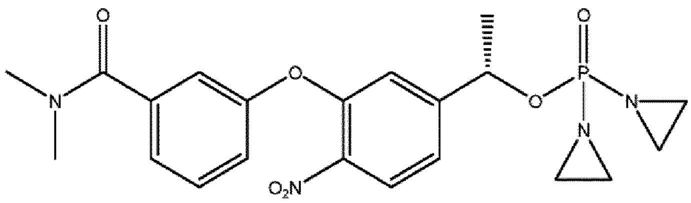

) has AKR1C3 enzyme inhibitory activity.

In vitro activity inhibition experiment of the compounds on AKR1C3

Experimental apparatus: Waters Acquity I Class UPLC Ultra Performance Liquid Chromatograph equipped with a Xevo G2-XS Q Tof HRMS Quadrupole Time-of-flight High-Resolution Mass Spectrometer

Buffers and materials:

1. PBS phosphate buffered saline solution,
2. PBS phosphate buffered saline solution of 20 mM NADPH
3. PBS phosphate buffered saline solution of 250 μg/mL AKR1C3
4. 50% $MeOH/H_2O$ solution of 250 μM AST-3424
5.50% $MeOH/H_2O$ solution of 250 μM progesterone
6. 100% acetonitrile solution of 1 μg/mL propranolol

Experimental procedures

Step 1, the reaction mixtures were put into Eppendorf tubes (microcentrifuge tubes) in quadruplicate (n=4) according to the table below and mixed gently.

| Materials | Negative controls (μL) | Samples (μL) |
|---|---|---|
| PBS | 68 | 58 |
| NADPH (20 mM) | 10 | 10 |
| AKR1C3 (250 μg/mL) | 10 | 10 |
| AST-3424 (250 μM) | 0 | 10 |

Step 2, the above mixtures were pre-incubated in duplicate at 37 °C for 30 minutes and 60 minutes.
Step 3, another 10 μL of PBS phosphate buffered saline solution of 20 mM NADPH and 2 μL of 50% MeOH/$H_2O$ solution of 250 μM progesterone were added to each Eppendorf tube and mixed gently.
Step 4, 50 μL of the mixture in the above step was immediately transferred to 100 μL of 100% acetonitrile solution of 1 μg/mL propranolol (internal standard (IS)).
Step 5, the remaining samples were incubated at 37°C for 30 minutes, and 100 μL of 100% acetonitrile solution of 1 μg/mL propranolol (internal standard (IS)) was added.
Step 6, 100 μL of reagent water was added to all the samples, vortexed at 1,100 rpm for 5 minutes, and centrifuged at 15,000 rpm for 10 minutes at room temperature. Step 7, all the samples were loaded on LC/MS to determine the content of reduced progesterone, namely 20α-dihydroprogesterone.
The test conditions for the LC-MS apparatus are shown below

| Items | Conditions |
|---|---|
| Apparatus: | Waters Acquity I Class Liquid Chromatograph |
| Chromatographic column: | Acquity UPLC BEHC18 Chromatographic Column (50*2.1 mm, 1.7 μm) |
| Flow rate: | 0.4 mL/min |
| Injection volume: | 3 μL |
| The composition of the mobile phase: | A: 0.1% (V/V) formic acid aqueous solution B: 0.1% (V/V) formic acid acetonitrile solution |
| The temperature of the column oven: | 40 °C |
| Detector: | Quadrupole Time-of-flight Mass Spectrometer Q-TOF MS |

The gradient of the liquid phase elution

| Time (min) | A (%) | B (%) |
|---|---|---|
| 0.00 | 90.0 | 0.0 |
| 1.5 | 5.0 | 95.0 |
| 2.00 | 5.0 | 95.0 |
| 2.30 | 90.0 | 10.0 |
| 3.00 | 90.0 | 10.0 |

Parameters of the Quadrupole Time-of-flight Mass Spectrometer

| Items | Parameters |
|---|---|
| Capillary kV | 2.5 |
| Sampling Cone V | 40 |
| Source temperature (°C) | 100 |
| Cone Gas (L/h) | 50 |
| Desolvation Gas (L/h) | 600 |
| Interface type | ES electron impact, Positive |
| Analyser mode | Sensitivity |
| Scan range | 50-800 m/z |

Step 9, the reduced progesterone (20α-dihydroprogesterone) is calculated: the peak area of the reduced progesterone, namley 20α-dihydroprogesterone and propranolol, in each sample was determined by LC/MS. The peak area ratios of reduced progesterone to propranolol (i.e., the ratios in the above table) were calculated, and when the

time is 0 the ratio is set to 0%.

AKR1C3 activity (%) = [(the amount of reduced progesterone after normalization of the sample) 30min - (the amount of reduced progesterone after normalization of the sample) 0 min] / [(the amount of reduced progesterone after normalization of the negative control group) 30min - (the amount of reduced progesterone after normalization of the negative control group) 0 min] * 100.

The AKR1C3 activity results in the above table were calculated according to the above formula.

Experimental results

| | Duration | | The peak areas of the liquid phase | | | | | | | | AKR1C3 activity % |
|---|---|---|---|---|---|---|---|---|---|---|---|
| | Incubation | Reaction | AST-3424 | Progesterone | | Reduced progesterone | | | Propranolol | | |
| | | | | Values | Averages | Values | Averages | Ratios* | Values | Averages | |
| Negative control groups | 30 min | 0 | Absent | 36658 | 36835 | 150 | 158 | 0.0034 | 51141 | 46879 | 0 |
| | | | | 37011 | | 166 | | | 42616 | | |
| | 60 min | | | 35244 | 35125 | 87 | 83 | 0.0019 | 42794 | 43650 | 0 |
| | | | | 35006 | | 79 | | | 44506 | | |
| | 30 min | 30 min | | 28675 | 26228 | 5616 | 6617 | 0.1506 | 43589 | 43935 | 100 |
| | | | | 23780 | | 7617 | | | 44280 | | |
| | 60 min | | | 25917 | 26492 | 6747 | 6051 | 0.1343 | 44258 | 45067 | 100 |
| | | | | 27067 | | 5354 | | | 45876 | | |
| Samples | 30 min | 0 | 154017 | 37107 | 37438 | 70 | 72 | 0.0017 | 43247 | 43436 | 0 |
| | | | 154843 | 37769 | | 74 | | | 43625 | | |
| | 60 min | | 108351 | 38050 | 37989 | 58 | 64 | 0.0015 | 43907 | 43697 | 0 |
| | | | 107508 | 37928 | | 70 | | | 43487 | | |
| | 30 min | 30 min | 104748 | 35187 | 35210 | 344 | 331 | 0.0074 | 45657 | 45051 | 3.9 |
| | | | 110630 | 35234 | | 319 | | | 44445 | | |
| | 60 min | | 52296 | 32636 | 32520 | 622 | 616 | 0.0137 | 45395 | 45101 | 9.2 |
| | | | 51309 | 32404 | | 610 | | | 44807 | | |

Analysis and summary of the experimental results

Table: The effect of AST-3424 on AKR1C3 activity

| | % AKR1C3 activity | |
|---|---|---|
| The time of incubation (min) | 0 μM AST-3424 | 25 μM AST-3424 |
| 30 | 100% | 3.9% |
| 60 | 100% | 9.2% |

The activity was 92.4 % when the test value of indometacin was at a concentration of 5um/L.

The effect of AST-3424 on the production of reduced progesterone: The aforementioned in vitro experiment proves that after pre-incubation for 30 minutes and 60 minutes, AST-3424 at a concentration of 25 μM basically inhibited

AKR1C3 activity: compared with the negative control groups, the production of the reduced progesterone, namely 20α-dihydroprogesterone, was reduced to 3.9% and 9.2%, respectively, proving that the compound AST-3424 are inhibitors of AKR1C3 enzyme.

**Content change experiment of prostaglandin in blood of cynomolgus monkeys before and after administration of AKR1C3 inhibitor AST-3424**

An experiment with three cynomolgus monkeys was performed according to the table below.

| Group | Amount | Administration | | | | | |
|---|---|---|---|---|---|---|---|
| | Male | Test substance | The dosage of the test substance (mg/kg) | The concentration of the solution of the test substance (mg/mL) | The volume of administrat ion (mL/kg) | Mode of administration | Collected Sample |
| 1 | 3(No. 101/ 102/103) | AST3424 | 1/Compound | 0.2 | 5 | Intravenous infusion | Plasma/ser um |

Four male cynomolgus monkeys were purchased from Guangxi Xiongsen Primate Development and Experiment Co., Ltd., and all of them were healthy cynomolgus monkeys that had passed the physical examination and have no abnormalities. Among them, three cynomolgus monkeys were used in the experiment of administration and the remaining one was used for preparing blank plasma.

Before administration, and 6, 24, 48 and 72 hours after the start of administration, 1 mL of blood was collected from the femoral vein or other suitable vein, and placed in a blood collection tube without anticoagulant. After collected, the blood sample was placed on ice and allowed to stand for 30-60 minutes before being centrifuged at 3,500 rpm for 10 minutes at 2-8°C to separate the serum. The collected serum was stored at -80°C before being analyzed.

Prostaglandin F2 in serum samples was analyzed by conventional ELISA methods. The measurement results are as follows.

The concentrations of prostaglandins E2 and F2 in the serum after single administration to cynomolgus monkeys by intravenous infusion

| Time point | The measured concentration of prostaglandin F2 (pg/ml) | | | | | |
|---|---|---|---|---|---|---|
| | 101 | | 102 | | 103 | |
| Before administration | 1828.51 | change and change percentage | 310.17 | change and change percentage | 1125.58 | change and change percentage |
| 6h | 165.32 | -1663.19, -90.95% | 260.80 | -49.37, -15.91% | 300.65 | -824.93, -73.29% |
| 24h | 816.63 | -1011.88, -55.34% | 3460.25 | 3150.08, 1000.15% | 4208.19 | 3082.61, +273.87% |
| 48h | 183.73 | -1644.78, -89.95% | 216.12 | -94.05, -30.32% | 541.05 | -584.53, -51.93% |
| 72h | 441.76 | -1386.75, -75.84% | 968.01 | 657.84, +212.09% | 1369.67 | 244.09, +21.68% |

Six hours after administration of 0.58 mg/kg of AST-3424 to cynomolgus monkeys, the content of prostaglandin F2a in all three cynomolgus monkeys decreased, indicating that the process of prostaglandin F2a secretion in cynomolgus monkeys can be inhibited after administration of AST-3424.

Obviously, it can be found that:

I: the levels of the content of prostaglandin F2a in blood of the three cynomolgus monkeys before using the interfering drugs have a significant difference, which were very different from each other.

II: Further, exploration of the content change of prostaglandin F2a in the three cynomolgus monkeys (101/102/103) after using infectious drugs shows that the contents of prostaglandin F2a of them are decreased within 6 hours while they are decreased or increased at 24, 48 and 72 hours after using AKR1C3 inhibitor (AST-3424), and this experimental fact shows that using an interfering drug (inhibitor) against AKR1C3 enzyme activity should be followed by the detection of change of prostaglandin within a suitable period of time, and for the three cynomolgus monkeys used in this experiment, 6 hours were appropriate.

III: Further, a comprehensive I/II exploration of the three cynomolgus monkeys for 6 hours after administration of the same amount of interfering drugs in the same intravenous injection, the absolute values of the content change of prostaglandin F2a were also completely different: the cynomolgus monkeys with highest content levels of prostaglandin F2a in their blood before administration of the interfering drugs also had the highest change values of prostaglandin F2a levels in their blood after administration of the interfering drug.

IV: Further, a comprehensive I/II exploration of the three cynomolgus monkeys for 6 hours after administration of the same amount of interfering drugs in the same intravenous injection, the change ratios of the content change of prostaglandin F2a were also completely different: the cynomolgus monkeys with highest content levels of prostaglandin F2a in their blood before administration of the interfering drugs also had the highest change ratios of the content of prostaglandin F2a levels in their blood after administration of the interfering drug.

In conclusion, a comprehensive exploration of the three cynomolgus monkeys without administration of interfering drugs and for 6 hours after administration of the same amount of interfering drugs in the same intravenous injection, the absolute value/relative change ratios of the content change of prostaglandin F2a was positively correlated with

PGF2a in cynomolgus monkeys without administration of interfering drug: the cynomolgus monkeys with highest content levels of prostaglandin F2a in their blood before administration of the interfering drugs also had the highest change value/relative change ratio of the content of prostaglandin F2a levels in their blood after administration of the interfering drug.

In fact, after using traditional immunohistochemical staining method (IHC) to detect AKR1C3 in the liver tissues obtained from these three Cynomolgus monkeys, it was found that the staining area of No.s 101 and 103 cynomolgus monkeys was higher than that of of No. 102 Cynomolgus monkey, i.e. the expression level of AKR1C3 enzyme in No. 101 and 103 Cynomolgus monkeys was higher than that of No. 102 Cynomolgus monkey.

The above in vitro and in vivo experiments confirm that route 1/2/3 is truly present, the conclusion by measuring the content of PGF2α and/or PGD2 and/or PGH2 or the content change and content change ratio before and after administration of interfering drugs in vitro and then combining with the data statistics can correlate the expression levels of AKR1C3 enzyme in biological samples, that is, it is clinically possible to directly obtain the expression levels of AKR1C3 enzyme of patients by measuring the content of prostaglandin, thereby screening a cancer or tumor patient with an appropriate expression level of AKR1C3 enzyme and administering the AKR1C3 enzyme-activated anti-cancer prodrug disclosed in the present invention to the patient.

## Claims

1. A method for measuring the expression level of AKR1C3 enzyme, comprising measuring the content of PGF2α and/or PGD2 and/or PGH2 to associate with the expression level of AKR1C3 enzyme in a biological sample.

2. A method for measuring the expression level of AKR1C3 enzyme, comprising measuring the content change of PGF2α and/or PGD2 and/or PGH2 before and after administration of an interfering drug to associate with the expression level of AKR1C3 enzyme in a biological sample.

3. A method for measuring the expression level of AKR1C3 enzyme, comprising measuring the content change rate of PGF2α and/or PGD2 and/or PGH2 before and after administration of an interfering drug to associate with the expression level of AKR1C3 enzyme in a biological sample.

4. The method for measuring according to claim 1, wherein the association refers to:

    when the content of PGF2α is in the range of A1, the expression level of AKR1C3 enzyme in the biological sample is high;
    when the content of PGF2α is in the range of B1, the expression level of AKR1C3 enzyme in the biological sample is medium;
    when the content of PGF2α is in the range of C1, the expression level of AKR1C3 enzyme in the biological sample is low;
    and/or
    when the content of PGD2 and/or PGH2 is in the range of A2, the expression level of AKR1C3 enzyme in the biological sample is low;
    when the content of PGD2 and/or PGH2 is in the range of B2, the expression level of AKR1C3 enzyme in the biological sample is medium;
    when the content of PGD2 and/or PGH2 is in the range of C2, the expression level of AKR1C3 enzyme in the biological sample is high;
    wherein the minimum value in the range of A1 is greater than or equal to the maximum value in the range of B1, the minimum value in the range B1 is greater than or equal to the maximum value in the range of C1,
    the minimum value in the range of A2 is greater than or equal to the maximum value in the range of B2, and the minimum value in the range of B2 is greater than or equal to the maximum value in the range of C2.

5. The method for measuring according to claim 2, wherein the association refers to:

    when the content change of PGF2α is in the range of a1, the expression level of AKR1C3 enzyme in biological sample is high;
    when the content change of PGF2α and/or PGD2 and/or PGH2 is in the range of b1, the expression level of AKR1C3 enzyme in biological sample is medium;
    when the content change of PGF2α and/or PGD2 and/or PGH2 is in the range of c1, the expression level of AKR1C3 enzyme in biological sample is low;

and/or
when the content change of PGD2 and/or PGH2 is in the range of a2, the expression level of AKR1C3 enzyme in biological sample is low;
when the content change of PGD2 and/or PGH2 is in the range of b2, the expression level of AKR1C3 enzyme in biological sample is medium;
when the content change of PGD2 and/or PGH2 is in the range of c2, the expression level of AKR1C3 enzyme in biological sample is high;
wherein the minimum value in the range of a1 is greater than or equal to the maximum value in the range of b1, the minimum value in the range of b1 is greater than or equal to the maximum value in the range of cl; the minimum value in the range of a2 is greater than or equal to the maximum value in the range of b2, and the minimum value in the of range b2 is greater than or equal to the maximum value in the range of c2.

**6.** The method for measuring according to claim 3, wherein the association refers to:

when the content change rate of PGF2α is in the range of α1, the expression level of AKR1C3 enzyme in biological sample is high;
when the content change rate of PGF2α is in the range of β1, the expression level of AKR1C3 enzyme in biological sample is medium;
when the content change rate of PGF2α is in the range of yl, the expression level of AKR1C3 enzyme in biological sample is low;
and/or
when the content change rate of PGD2 and/or PGH2 is in the range of α2, the expression level of AKR1C3 enzyme in biological sample is low;
when the content change rate of PGD2 and/or PGH2 is in the range of β2, the expression level of AKR1C3 enzyme in biological sample is medium;
when the content change rate of PGD2 and/or PGH2 is in the range of γ2, the expression level of AKR1C3 enzyme in biological sample is high;
wherein the minimum value in the range of α1 is greater than or equal to the maximum value in the range of β1, the minimum value in the range of β1 is greater than or equal to the maximum value in the range of γ1, the minimum value in the range of α2 is greater than or equal to the maximum value in the range β2, and the minimum value in the range of β2 is greater than or equal to the maximum value in the range of γ2.

**7.** The method for measuring according to claim 2 or 3, wherein the interference drug is an AKR1C3 enzyme inhibitor or an AKR1C3 enzyme agonist.

**8.** The method for measuring according to claim 7, wherein the AKR1C3 enzyme inhibitor is

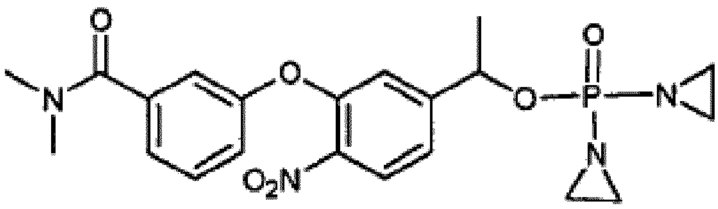

;

or

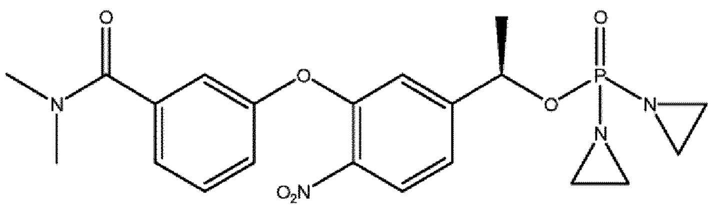

;

or

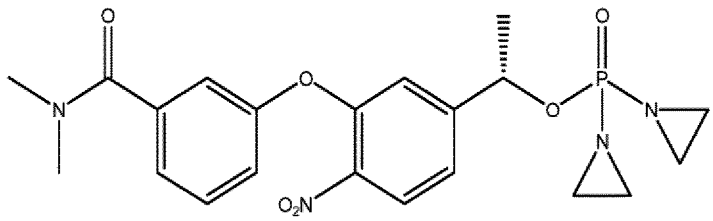

; or Indomethacin.

**9.** The method for measuring according to claim 1, 2 or 3, wherein the biological sample is blood or serum.

**10.** The method for measuring according to claim 2 or 3, comprising the following operations:

measuring the content of PGF2$\alpha$ and/or PGD2 and/or PGH2 in a living body, a living biological organ or a living biological tissue;
administering the interfering drug to the living body, the living biological organ or the living biological tissue;
measuring the content of PGF2$\alpha$ and/or PGD2 and/or PGH2 in the living body, the living biological organ or the living biological tissue after the administration of the interfering drug;
calculating the content change and the content change rate before and after administration of the interfering drug, and obtanining the expression level of AKR1C3 enzyme in biological sample according to the corresponding relationship.

**11.** A method for screening and administrating to a patients with cancer, tumor or a disorder caused by cancer or tumor, or a cell proliferative disease, comprising administering an AKR1C3 enzyme-activated anti-cancer prodrug to the patient after the expression level of AKR1C3 enzyme is obtained by using the method for measuring the expression level of AKR1C3 enzyme according to any of the preceding claims 1 to 10.

**12.** A method for screening and administrating to a patient with cancer, tumor or a disorder caused by cancer or tumor or a cell proliferative disease, comprising administering an AKR1C3 enzyme-activated anti-cancer prodrug to the patient when the level of enzyme is high after the expression level of AKR1C3 enzyme is obtained by using the method for measuring the expression level of AKR1C3 enzyme according to any of the preceding claims 1 to 10.

**13.** A method for screening and administrating to a patient with cancer, tumor or a disorder caused by cancer or tumor or a cell proliferative disease, comprising administering AKR1C3 enzyme-activated anti-cancer prodrug to the patient when the level of enzyme is high or medium after the expression level of AKR1C3 enzyme is obtained by using the method for measuring the expression level of AKR1C3 enzyme according to any of the preceding claims 1 to 10.

**14.** The method for administrating according to claim 11, 12 or 13, wherein the AKR1C3 enzyme-activated anti-cancer prodrug contains the compound of structural formula I:

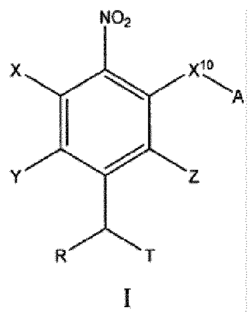

I

wherein,

$X^{10}$ is O, S, SO or $SO_2$;
A is $C_6$-$C_{10}$ aryl or substituted aryl, 5-15 membered heteroaryl or substituted heteroaryl or -N=CR$^1$R$^2$; wherein R$^1$ and R$^2$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

X, Y and Z are each independently hydrogen, CN, halogen, $C_1$-$C_6$ alkyl, $C_2$-$C_6$ alkynyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

$R^{13}$ and $R^{14}$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl or ether, or $R^{13}$ and $R^{14}$ groups together with the nitrogen atoms to which they are bonded form 5-7 membered heterocycle;

T comprisess an amino phosphate alkylating agent, i.e., T is -L-D, and includes the following six cases:

-D is $-P(Z^1)(Z^5-X^5-Y^5)_n$, $Z^1$ is O or S, $Z^5$ is N, S or O, $X^5$ is an optionally substituted ethylidene, $Y^5$ is a halogen atom or $-OSO_2-R^{20}$, $R^{20}$ is optionally substituted hydrocarbyl, aryl, cycloalkyl, heterocycly or heteroaryl, n is 1 or 2, L is selected from -O-, -S-, -OCOO-, $-NR^6CO-$, -OCO-, $-NR^6SO_2-$, $-OCONR^6-$, quaternary ammonium, sulfonate group $-OSO_2-$;

or

$Z^1$ is O or S, $Z^5-X^5-Y^5$ is an aziridinyl-$NCH_2CH_2$ moiety;

or

-L- is -O-, -D is $-P(Z^1)(Z^5-X^5-Y^5)_n$, $Z^1$ is O or S, $Z^5$ is N, S or O, $X^5$ is an optionally substituted ethylidene, $Y^5$ is a halogen atom or $-OSO_2-R^{20}$, $R^{20}$ is an optionally substituted hydrocarbyl, aryl, cycloalkyl, heterocyclyl or heteroaryl, and n is 1 or 2;

or

-L- is -O-, $Z^1$ is O or S, and $Z^5-X^5-Y^5$ is an aziridinyl $-NCH_2CH_2$ moiety;

or

-L-D is $-OP(Z^1)(NR^{30}CH_2CH_2Cl)_2$, $-OP(Z^1)(NR^{30}CH_2CH_2Br)_2$, $-OP(Z^1)(NR^{30}_2)(N(CH_2CH_2X^1)_2)$, $-OP(Z^1)(N(CH_2)_2)_2$, or $-OP(Z^1)(N(CH_2CH_2Cl)_2)_2$, wherein each $R^{30}$ is each independently H, $C_1$-$C_6$ hydrocarbyl or two $R^{30}$ groups together with the nitrogen atoms to which they are linked form 5-7 membered heterocycle, $Z^1$ is O or S, $X^1$ is Cl, Br or $-OSO_2Me$;

or

-L-D is $-OP(Z^1)(NHCH_2CH_2Cl)_2$, $-OP(Z^1)(NHCH_2CH_2Br)_2$, $-OP(Z^1)(NH_2)(N(CH_2CH_2X^1)_2)$, $-OP(Z^1)(N(CH_2)_2)_2$, or $-OP(Z^1)(N(CH_2CH_2Cl)_2)_2$, and $X^1$ is Cl, Br or $-OSO_2Me$;

and the alkyl, alkenyl, alkynyl, cycloalkyl, aryl, heterocycle, heteroaryl, and the ether group are substituted or unsubstituted.

**15.** The method for administrating according to claim 11, 12 or 13, wherein the AKR1C3 enzyme-activated anti-cancer prodrug contains a compound having a structural formula selected from the group consisting of:

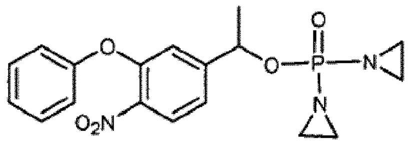,
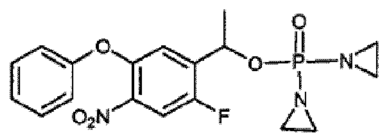,
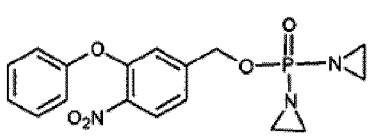,
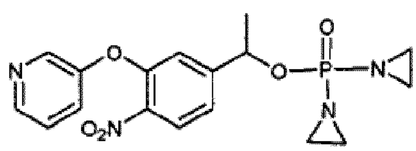,
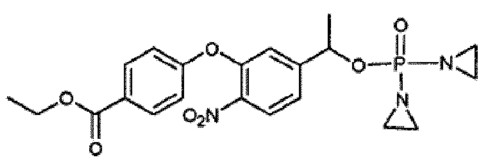,
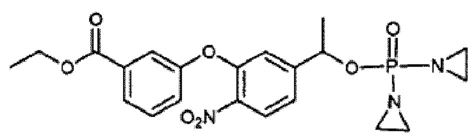,

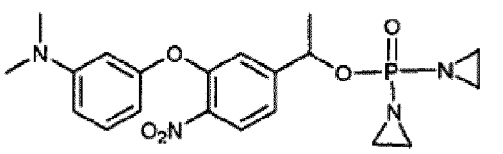 , 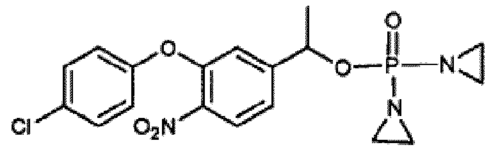 ,

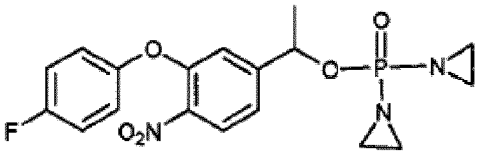 , 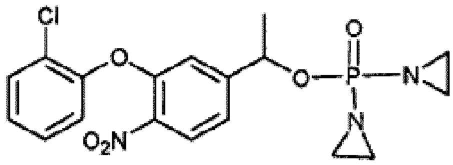 ,

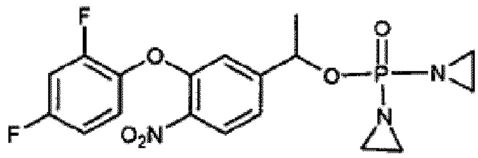 , 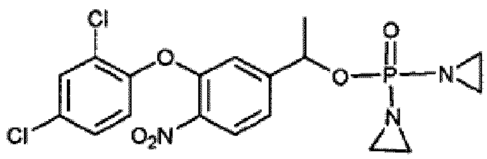 ,

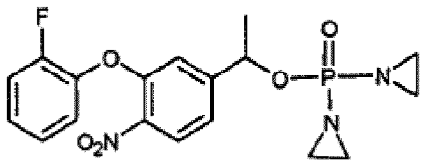 , 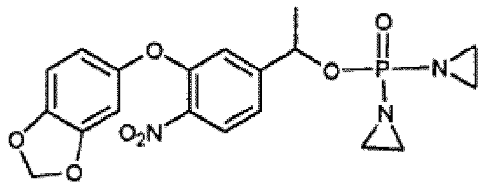 ,

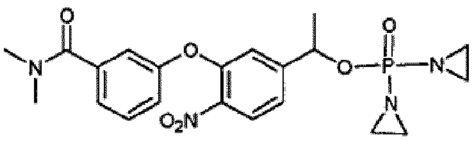 , 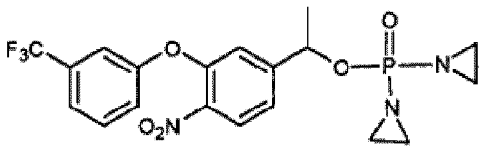 ,

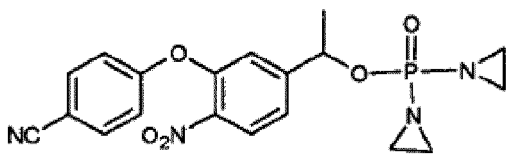 , 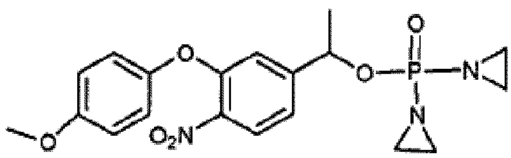 ,

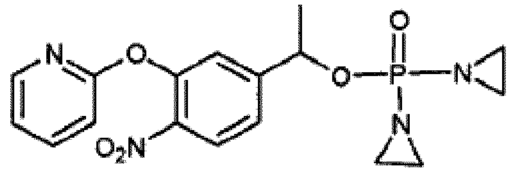 , 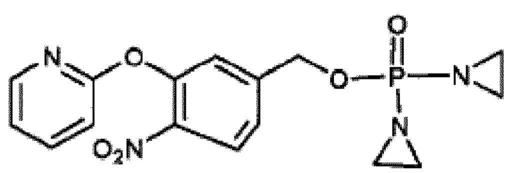 ,

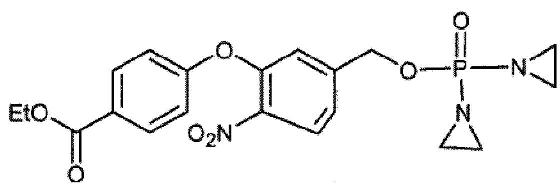 , 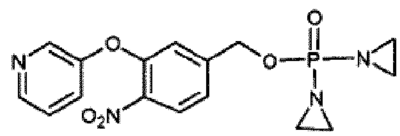 ,

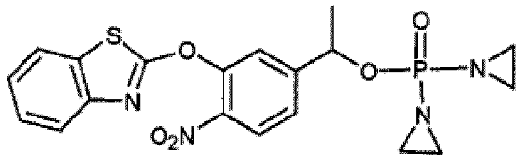,
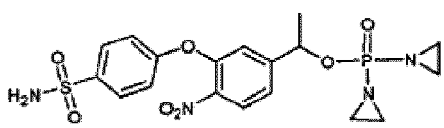,

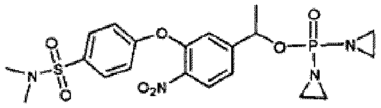,
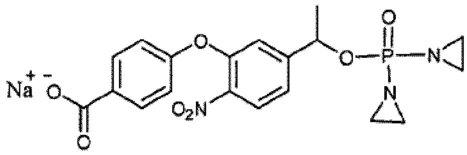,

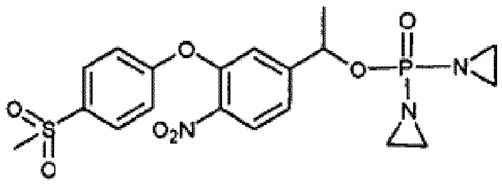,
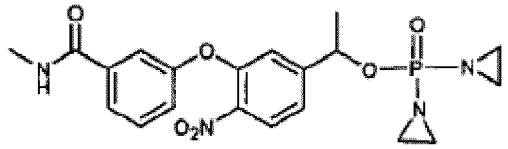,

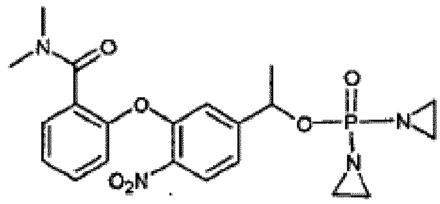,
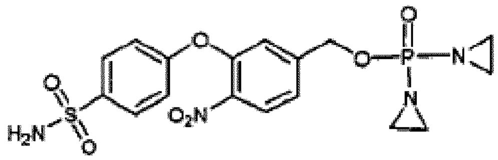,

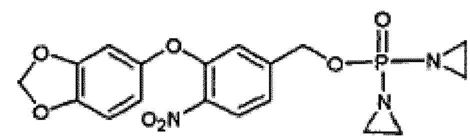,
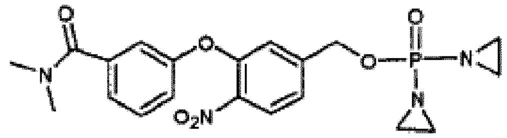,

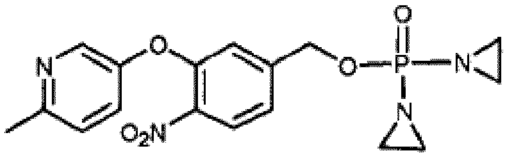,
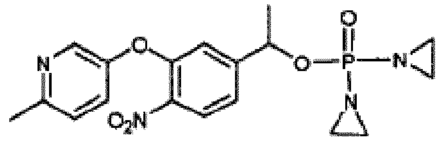,

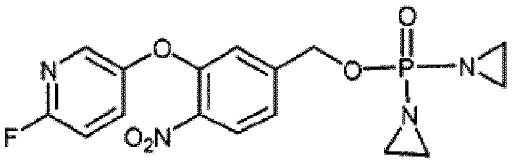,
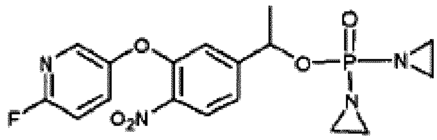,

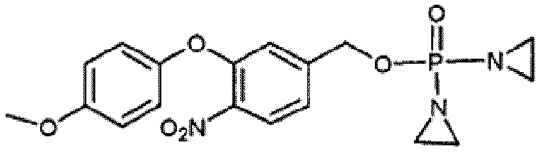,
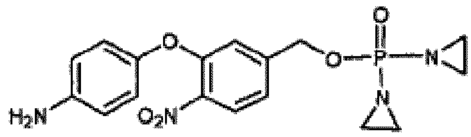,

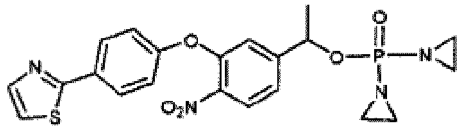,
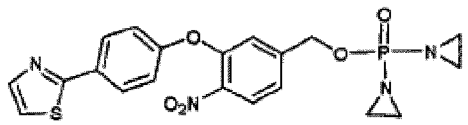,

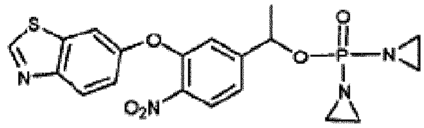,
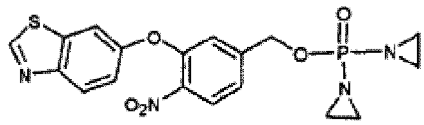,

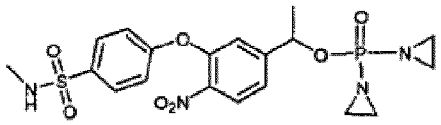,
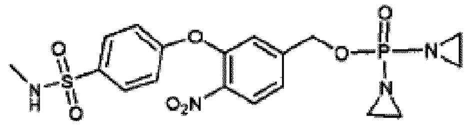,

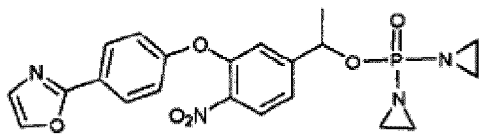,
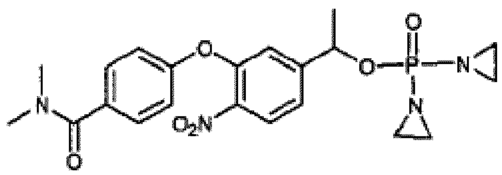,

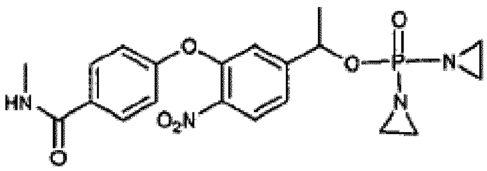,
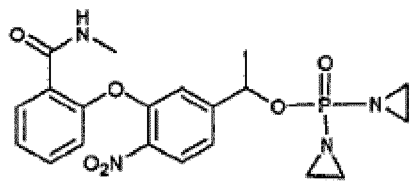,

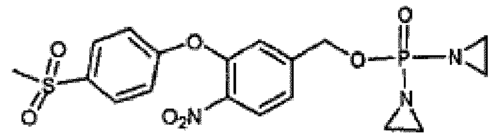,
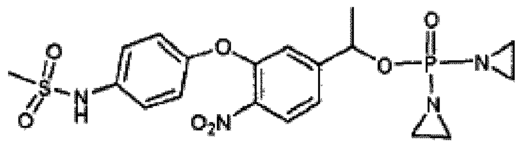,

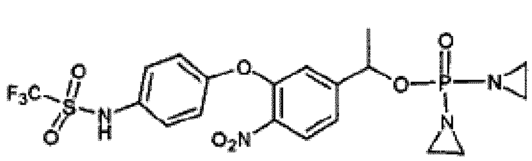,
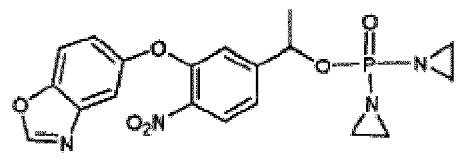,

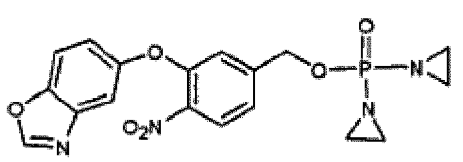,
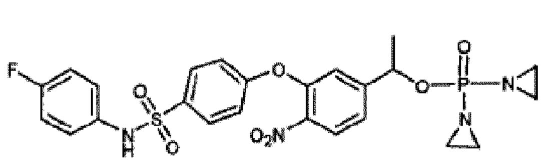,

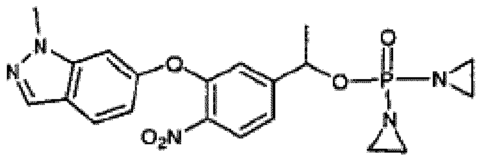 , 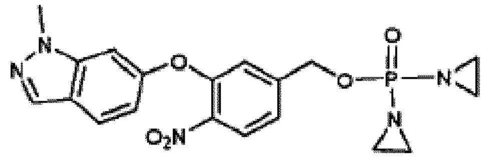 ,

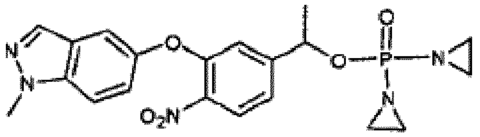 , 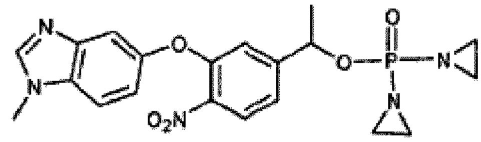 ,

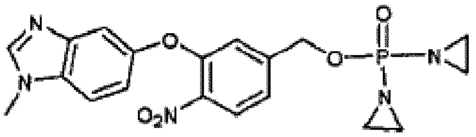 , 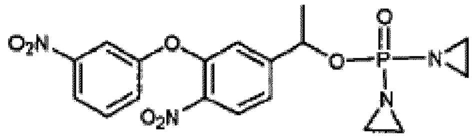 ,

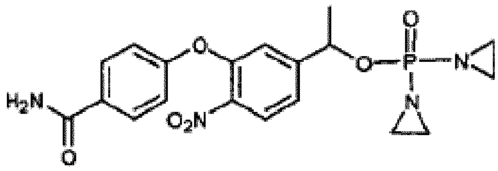 , 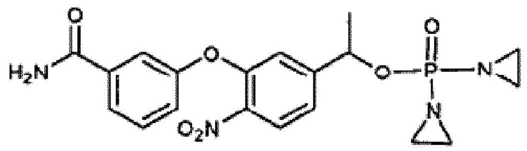 ,

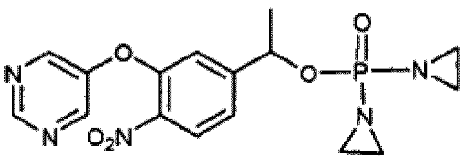 , 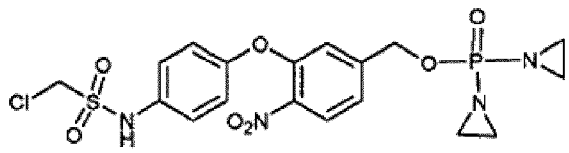 ,

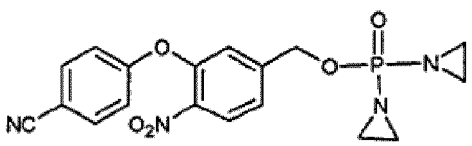 , 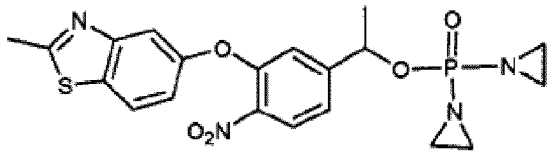 ,

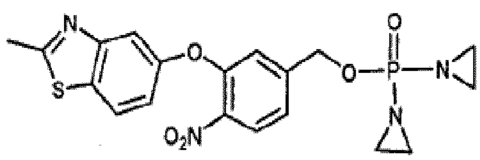 , 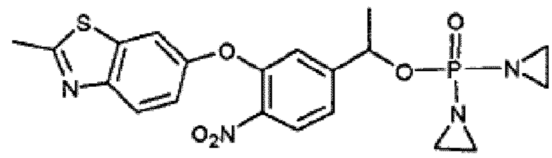 ,

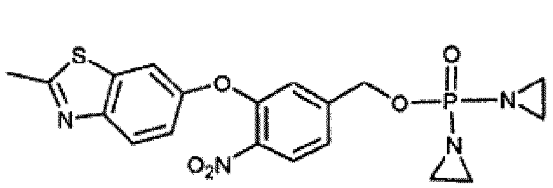 , 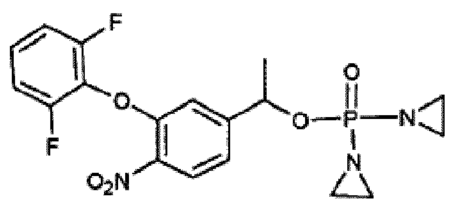 ,

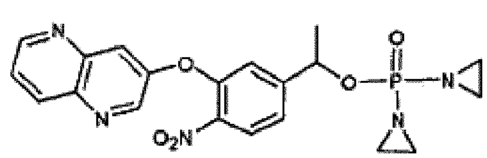,
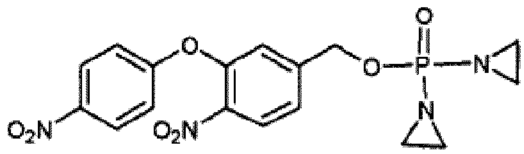,

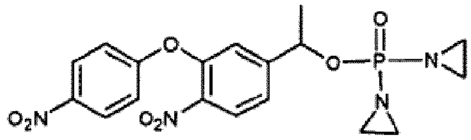,
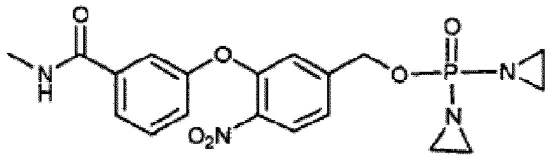,

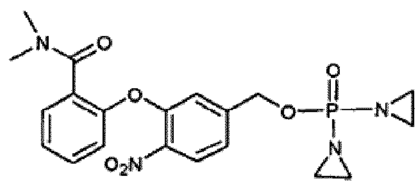,
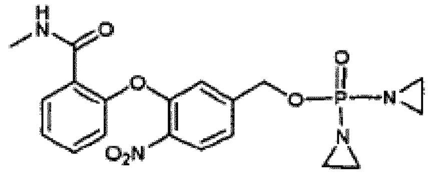,

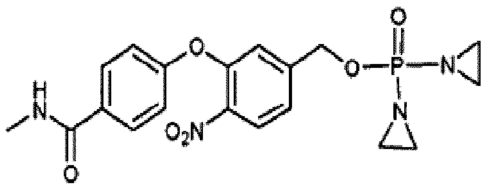,
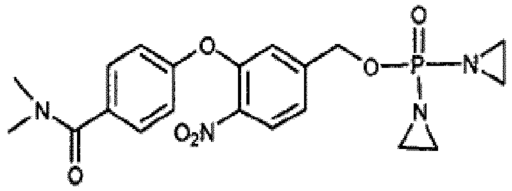,

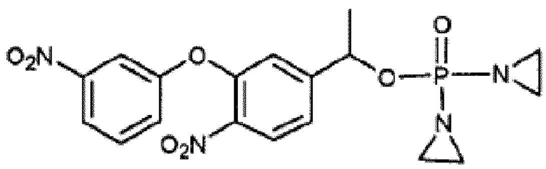,
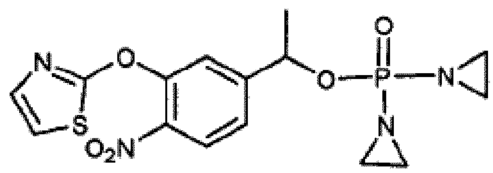,

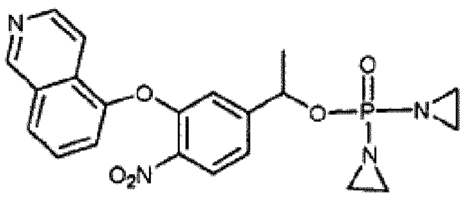,
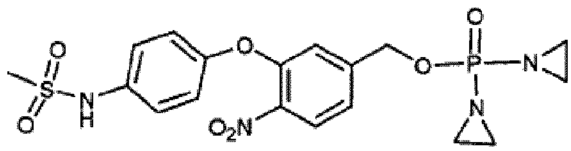,

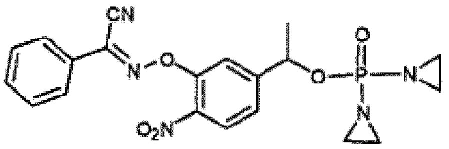,
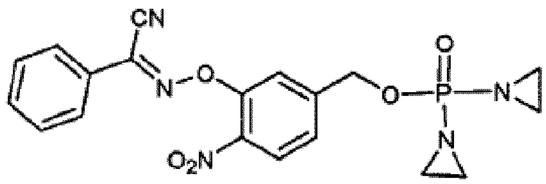,

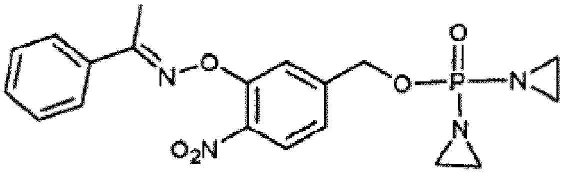
,
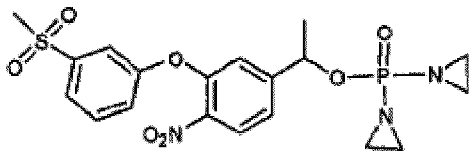
,

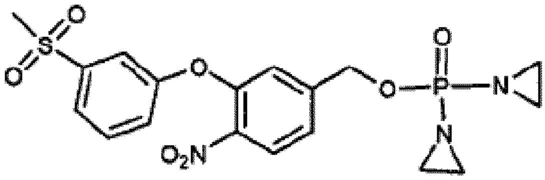
,
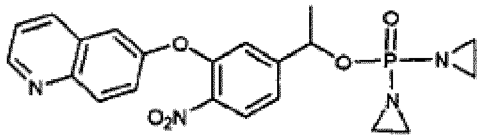
,

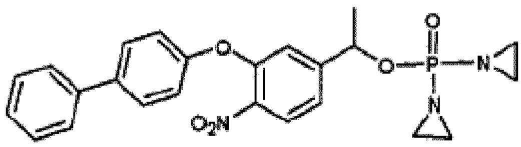
,
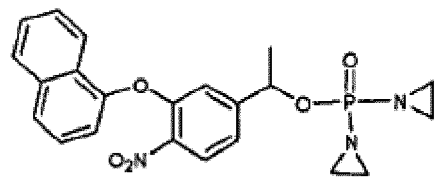
,

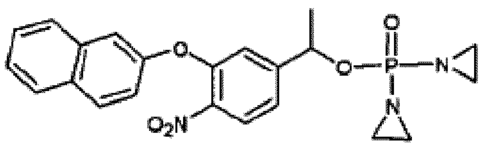
,
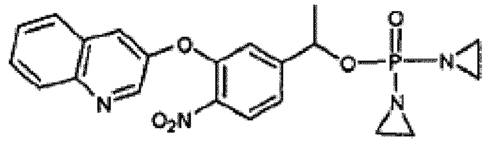
,

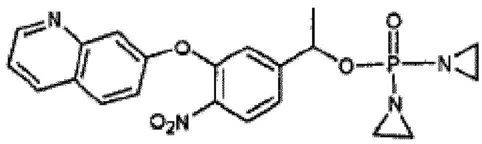
,
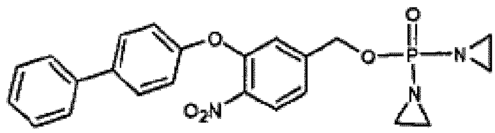
,

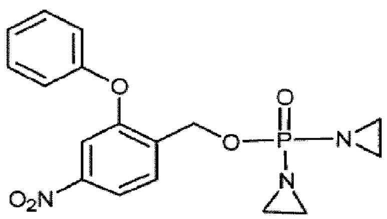
,
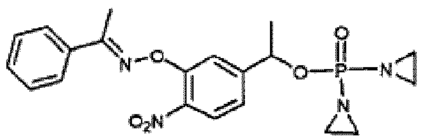
,

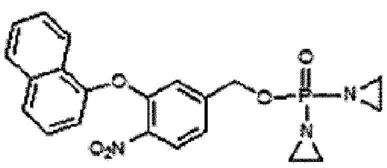
,
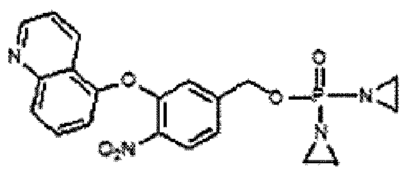
,

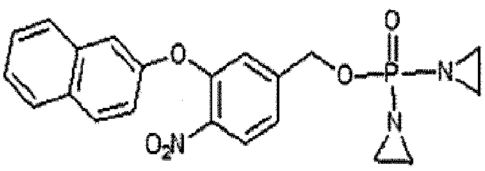,
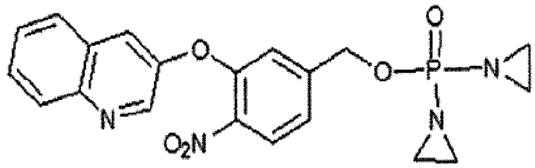,

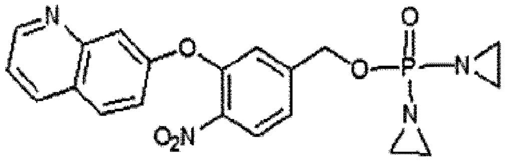,
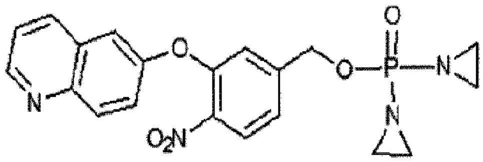,

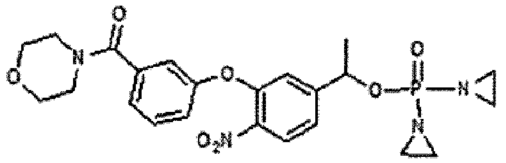,
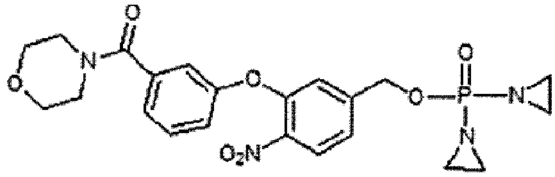,

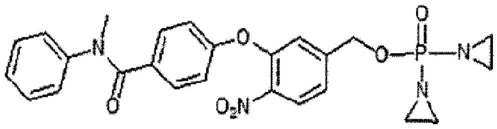,
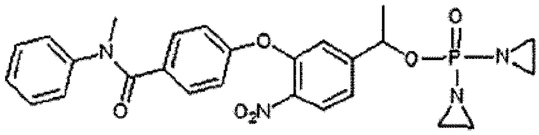,

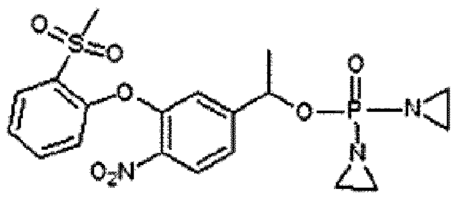,
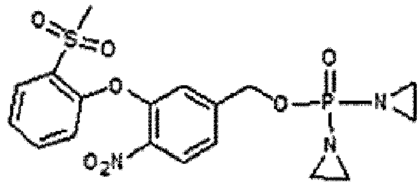,

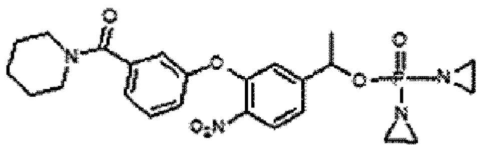,
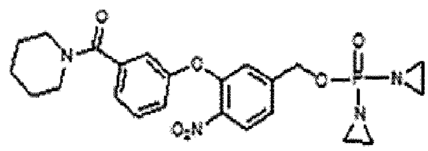,

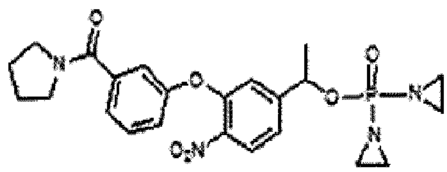.
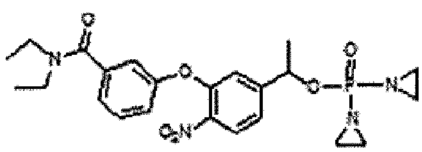.

, ,

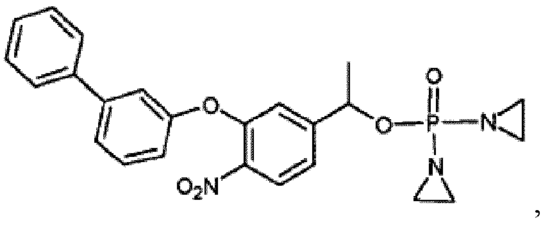

,

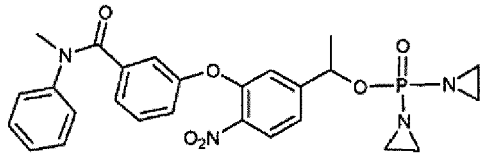

,

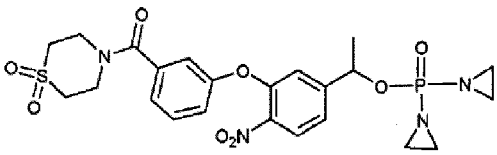

,

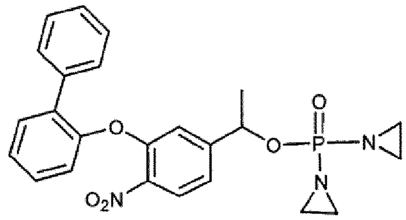

,

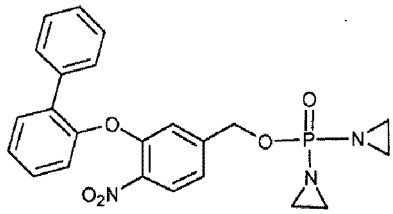

,

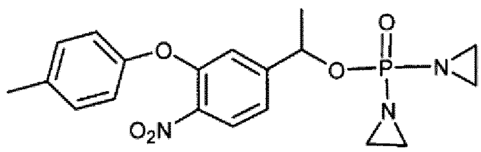

,

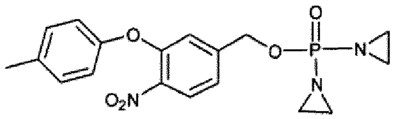

,

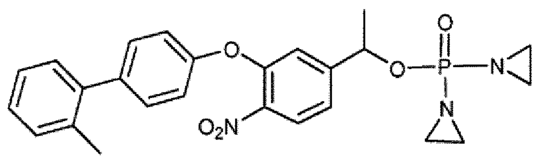

,

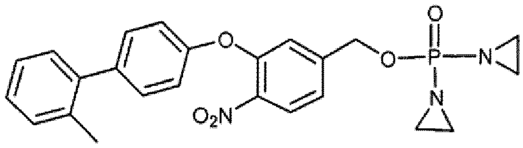

,

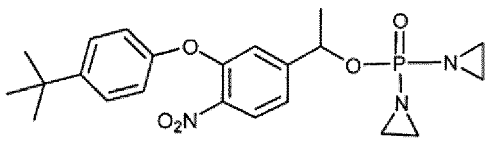

,

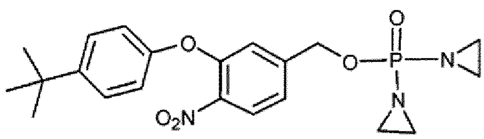

,

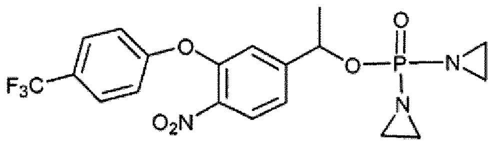

,

, 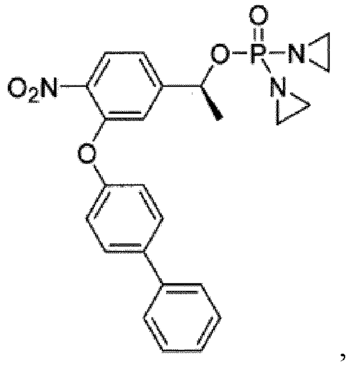 , 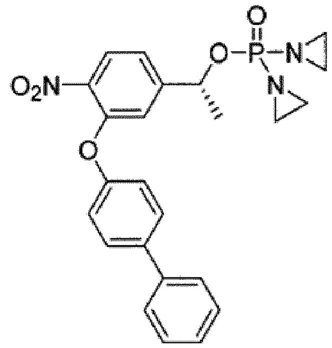 ,

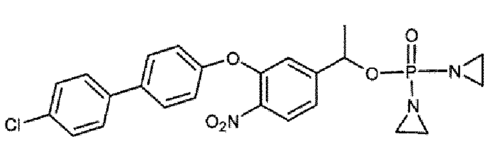 , 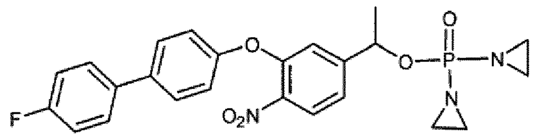 ,

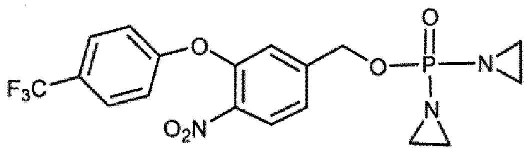

and

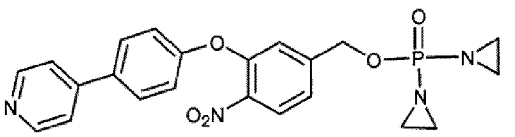 .

**16.** The method for administrating according to claim 11, 12 or 13, wherein the AKR1C3 enzyme-activated anti-cancer prodrug contains a compound of structural formula II:

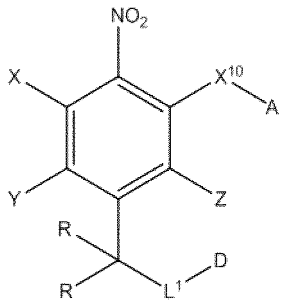

II

wherein,

$X^{10}$ is O, S, SO or $SO_2$;

A is $C_6$-$C_{10}$ aryl or substituted aryl, 5-15 membered heteroaryl or substituted heteroaryl or $-N=CR^1R^2$; wherein $R^1$ and $R^2$ are each independently hydrogen, $C_1$-$C_6$ alkyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

X, Y and Z are each independently hydrogen, CN, halogeno, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, $-CONR^{13}R^{14}$ or $-NR^{13}COR^{14}$;

each R is independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl, ether, -$CONR^{13}R^{14}$ or -$NR^{13}COR^{14}$;
$R^{13}$ and $R^{14}$ are each independently hydrogen, $C_1$-$C_6$ alkyl, $C_3$-$C_8$ cycloalkyl, $C_6$-$C_{10}$ aryl, 4-15 membered heterlcycle, 5-15 membered heteroaryl or ether, or $R^{13}$ and $R^{14}$ together with the nitrogen atom to which they are bonded form 5-7 membered heterocyclyl;
$L^1$ and D are as defined in the description, and the specific definitions are as follows:

$L^1$ is selected from:

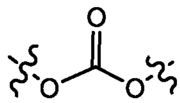 , 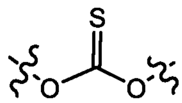 , 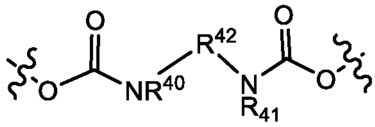 , 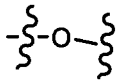 ,

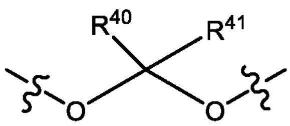 and 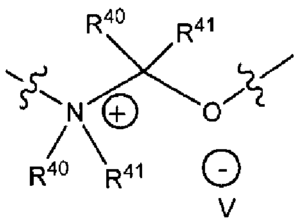 ;

wherein $R^{40}$ and $R^{41}$ are independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15 membered heterocycle, 5-15 membered heteroaryl;
$R^{42}$ is a $C_2$-$C_3$ alkylene or heteroalkylene optionally substituted with 1-3 $C_1$-$C_6$ alkyl; V(-) is any anion, preferably a pharmaceutically acceptable anion, and
D is a moiety that makes D-OH an anti-cancer drug, wherein OH is an aliphatic hydroxyl or phenolic hydroxyl; in other words, D is a group after hydroxyl of the anti-cancer drug D-OH is removed;
or
$L^1$ is

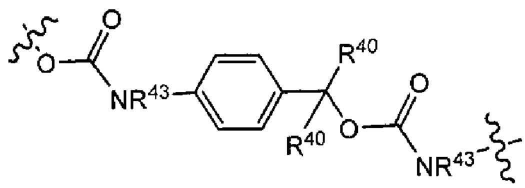 , 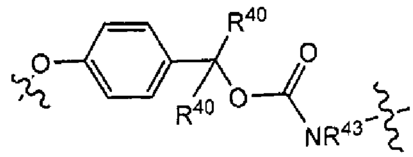

or

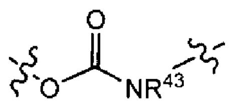 ;

wherein $R^{40}$ is as defined above, $R^{43}$ is hydrogen or together with D forms heterocycle, and the phenyl moiety is optionally substituted; and
D is a moiety that makes D-$NR^{43}$H an anti-cancer drug; in other words, D is a group after amino or amine of the anti-cancer drug D-$NR^{43}$H is removed;
or
$L^1$ is bond, -O-C($R^{40}R^{41}$)-, -O-C($R^{40}R^{41}$)-$NR^{40}R^{41}$(+)-C($R^{40}R^{41}$)- or

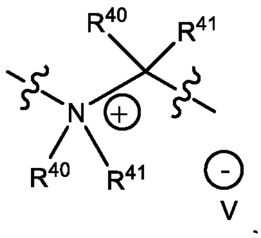

wherein $R^{40}$, $R^{41}$ and V are as as defined above; and

D is an anti-cancer drug containing a primary or secondary amine, wherein the primary or secondary amine is bonded to $L^1$; and

the alkyl, alkenyl, alkynyl, cycloalkyl, aryl, heterocycle, heteroaryl, and the ether group are substituted or unsubstituted.

**17.** The method for administrating according to claim 16, wherein in the compound of structural formula II contained in the AKR1C3 enzyme-activated anti-cancer prodrug,

D-OH is selected from the following anti-cancer drugs containing an -OH group: gemcitabine, estramusting, pudnimnstine, chlorozotocin, ranimustine, mannomustine, mitobronitol, dibromodulcitol, aclacinomycins, anthramycin, bleomycin, carubicin, carzinophilin, chromomycin, actinomycin D, daunorubicin, mycophenolic acid, nogalamycin, olivomycin, peplomycin, plicamycin, puromycin, streptonigrin, streptozocin, tubercidin, ubenimex, zinostatin, zorubicin, denopterin, fludarabine, ancitabine, azacitidine, 6-azauridine, cytarabine, dideoxyuridine, doxifluridine, enocitabine, floxuridine, L-asparaginase, pulmozyme, aceglatone, elliptinium acetate, etoglucid, interferon-alpha, interferon-beta, interferon-gamma, interleukin-2, lentinan, mitoxantrone, mopidamol, pentostatin, pirarubicin, podophyllinic acid, sizofiran, paclitaxel, teniposide, tenuazonic acid, vinblastine, vincristine;

D-$NR^{43}$H is selected from the following anti-cancer drugs: erlotinib, meturedepa, uredepa, imatinib, trimethylolomelamine, gefitinib, uracil mustard, carmustine, chlorozotocin, fotemustine, nimustine, ranimustine, dacarbazine, mannomustine, actinomycin, anthramycin, bleomycin, actinomycin C, carubicin, carzinophilin, actinomycin D, peplomycin, puromycin, streptozocin, ubenimex, zinostatin, denopterin, pteropterin, trimetrexate, 6-mercaptopurine, thiamiprine, thioguanine, 6-azauridine, carmofur, dideoxyuridine, doxifluridine, enocitabine, floxuridine, 5-fluorouracil, tegafur, L-asparaginase, pulmozyme, amsacrine, bisantrene, demecolcine, diaziquone, elliptinium acetate, flutamide, hydroxyurea, interferon-alpha, interferon-beta, interferon-gamma, interleukin-2, mitoxantrone, nitracrine, pentostatin, phenamet, 2-ethylhydrazide, procarbazine, razoxane, erlotonib, urethane, vinblastine, vincristine;

the anti-cancer drugs containing a tertiary or secondary nitrogen atom is selected from altretamine, triethylenemelamine, chlorambuci, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, pipobroman, actinomycin, anthramycin, carzinophilin, actinomycin D, nogalamycin, porfiromycin, puromycin, streptozocin, tubercidin, fludarabine, ancitabine, azacitidine, cytarabine, dideoxyuridine, enocitabine, floxuridine, L-asparaginase, pulmozyme, aldophosphamide glycoside, bestrabucil, diaziquone, interferon-alpha, interferon-beta, interferon-gamma, interleukin-2, mitoguazone, mopidamol, nitracrine, pentostatin, phenamet, razoxane, spirogermanium, tamoxifen, triaziquone, 2,2',2"-trichlorotriethylamine, vinblastine, vincristine.

**18.** The method for administrating according to claim 11, 12 or 13, wherein the AKR1C3 enzyme-activated anti-cancer prodrug contains a compound with a structural formula selected from the group consisting of:

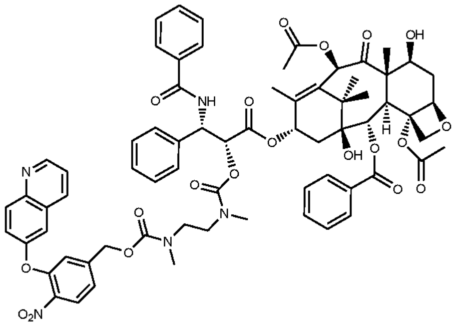
,
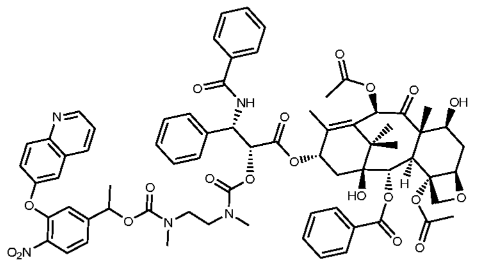
,

or

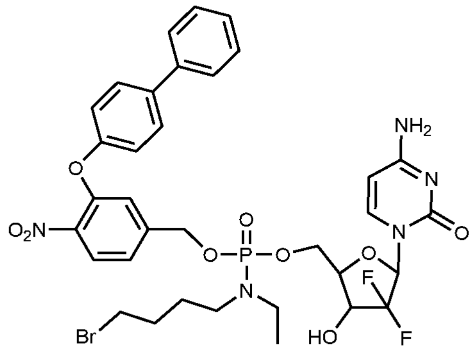
.

**19.** An assembly for measuring the expression level of AKR1C3 enzyme, comprising:

a component that contacts and reacts with the biological sample, and quantitatively or semi-quantitatively associates with the content of PGF2α and/or PGD2 and/or PGH2 in the biological sample according to the signal of the reaction;
a control comparison component that is used to compare the signal of reaction to comparatively compare and obtain the expression level of AKR1C3 enzyme corresponding to the content of PGF2α and/or PGD2 and/or PGH2, the content change of PGF2α and/or PGD2 and/or PGH2 before and after administration of the interfering drug, or the content change rate of PGF2α and/or PGD2 and/or PGH2 before and after administration of the interfering drug in the biological sample.

**20.** An administration device, comprising:

the assembly for measuring the expression level of AKR1C3 enzyme according to claim 19;
an administration assembly, which contains an AKR1C3 enzyme-activated anti-cancer prodrug.

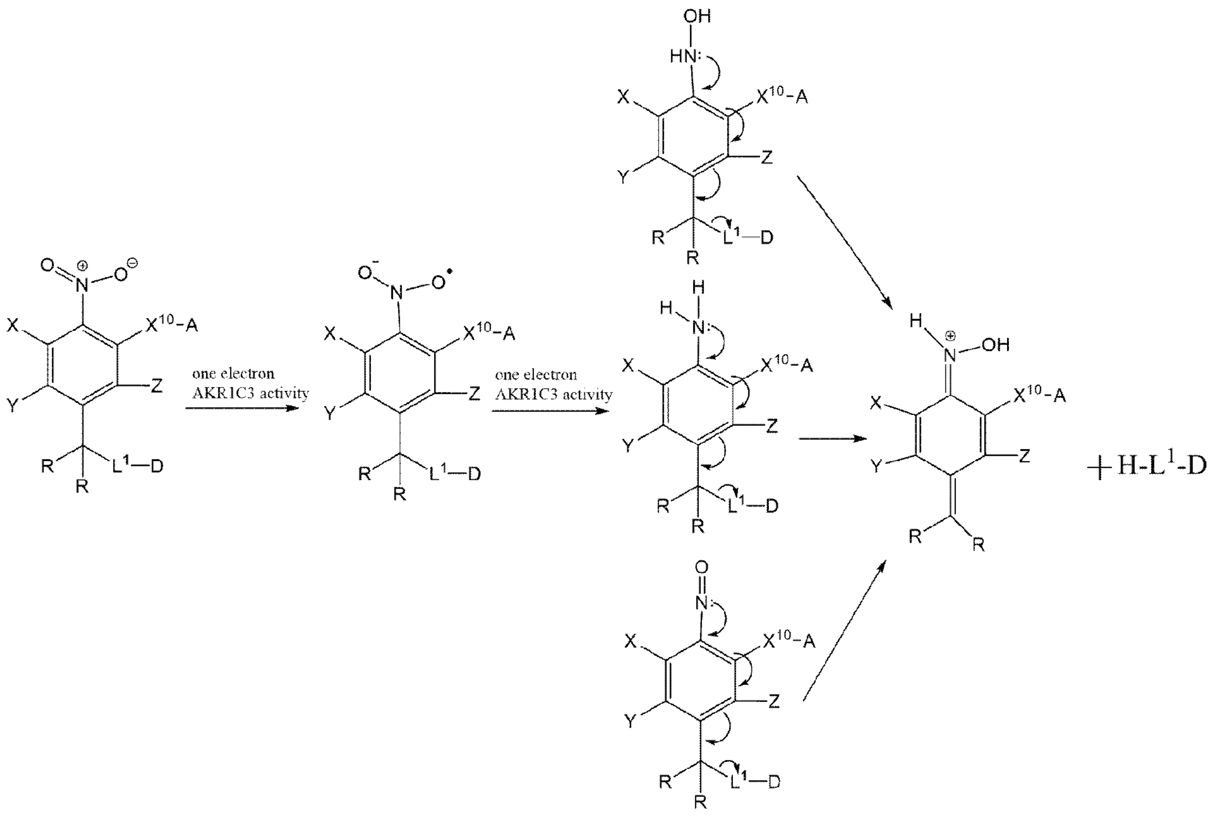


A B C D

FIG.1

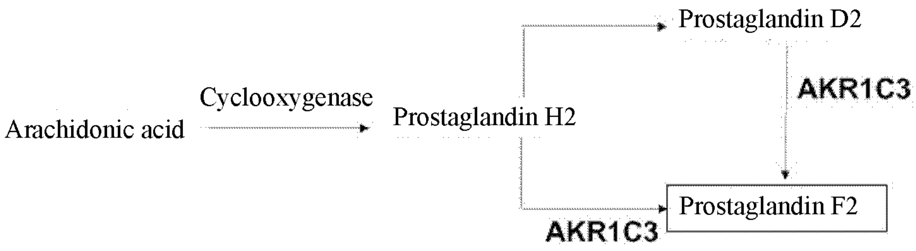


Route 1

FIG.2

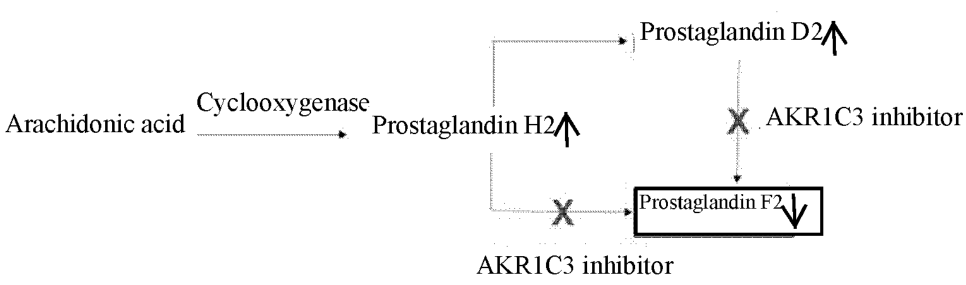


Route 2

FIG.3

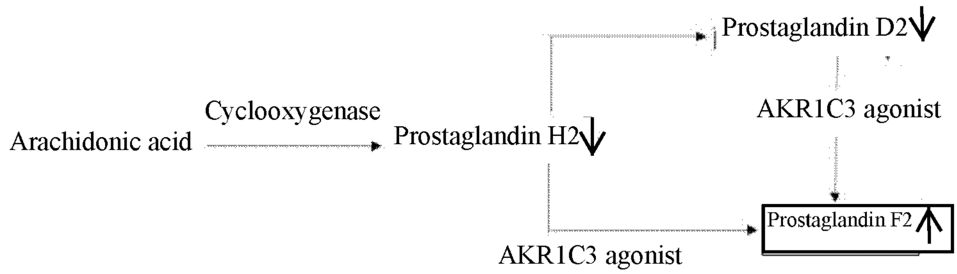
Prostaglandin D2
Cyclooxygenase
AKR1C3 agonist
Arachidonic acid
Prostaglandin H2
Prostaglandin F2
AKR1C3 agonist

Route 4

FIG.4

**INTERNATIONAL SEARCH REPORT**

International application No.
**PCT/CN2020/133538**

**A. CLASSIFICATION OF SUBJECT MATTER**

G01N 33/50(2006.01)i

According to International Patent Classification (IPC) or to both national classification and IPC

**B. FIELDS SEARCHED**

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

CNPAT, CNKI, WPI, EPODOC: 艾欣达伟, 段建新, 谢燕彬, 姬金凤, 醛酮还原酶, 前列腺素, 表达, 水平, 活性, 活力, 抑制剂, 激动剂, AKR1C3, hydroxysteroid dehydrogenase, prostaglandin, prostaglandin D2 11-ketoreductase, PGH2, PGD2, PGF2, PGF2α, express+, level?, activit+, inhibit+, agonist+

**C. DOCUMENTS CONSIDERED TO BE RELEVANT**

| Category* | Citation of document, with indication, where appropriate, of the relevant passages | Relevant to claim No. |
|---|---|---|
| Y | DOZIER, Brandy L. et al. "Two Pathways for Prostaglandin F2α (PGF2α) Synthesis by the Primate Periovulatory Follicle" *Reproduction,* Vol. 136, No. 1, 04 April 2008 (2008-04-04), ISSN: 1741-7899, the abstract, p. 5, paragraph 4, and figures 1 and 6 | 1-3, 7-10, 19, 20 |
| Y | CN 108290911 A (SHENZHEN THRESHOLD PHARMACEUTICALS, INC.) 17 July 2018 (2018-07-17) claims 1-14, and description, paragraphs [0007]-[0013] | 1-3, 7-10, 19, 20 |
| A | CN 102836433 A (PEKING UNIVERSITY et al.) 26 December 2012 (2012-12-26) entire document | 1-10, 19, 20 |
| A | CN 109833321 A (DALIAN INSTITUTE OF CHEMICAL PHYSICS, CHINESE ACADEMY OF SCIENCES) 04 June 2019 (2019-06-04) entire document | 1-10, 19, 20 |
| A | WO 2010044686 A1 (AUCKLAND UNISERVICES LIMITED) 22 April 2010 (2010-04-22) entire document | 1-10, 19, 20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2021** | **25 February 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/133538**

**C. DOCUMENTS CONSIDERED TO BE RELEVANT**

| Category* | Citation of document, with indication, where appropriate, of the relevant passages | Relevant to claim No. |
|---|---|---|
| A | WO 2018148721 A1 (TEXAS TECHNOLOGY UNIVERSITY SYSTEM et al.) 16 August 2018 (2018-08-16)<br>entire document | 1-10, 19, 20 |
| A | WO 2019002015 A1 (BAYER PHARMA AKTIENGESELLSCHAFT) 03 January 2019 (2019-01-03)<br>entire document | 1-10, 19, 20 |

Form PCT/ISA/210 (second sheet) (January 2015)

**INTERNATIONAL SEARCH REPORT**

International application No.
**PCT/CN2020/133538**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11-18**
because they relate to subject matter not required to be searched by this Authority, namely:

[1] A method for treatment of the human or animal body by means of surgery or therapy as stipulated in PCT Rule 39.1(4). Specifically, claims 11-18 set forth a method for screening and administering patients with cancer, tumors, or diseases caused by cancer, tumors, or cell proliferative diseases, including administering to patients an anti-cancer prodrug activated by the AKR1C3 enzyme. It can be seen that, it includes the steps of administering drugs to living organisms to treat cancer. Therefore, the technical solutions set forth by claims 11-18 relate to methods for treatment of the human or animal body by surgery or therapy, as well as diagnostic methods, as in PCT Rule 39.1(4).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/133538**

| Patent document cited in search report | | | Publication date (day/month/year) | Patent family member(s) | | | Publication date (day/month/year) |
|---|---|---|---|---|---|---|---|
| CN | 108290911 | A | 17 July 2018 | CN | 108290911 | B | 08 May 2020 |
| | | | | BR | 112017025778 | A2 | 14 August 2018 |
| | | | | TW | 201726695 | A | 01 August 2017 |
| | | | | CA | 2990696 | A1 | 26 May 2017 |
| | | | | ES | 2781398 | T3 | 01 September 2020 |
| | | | | KR | 101985778 | B1 | 04 June 2019 |
| | | | | IL | 256569 | D0 | 28 February 2018 |
| | | | | EP | 3390415 | A1 | 24 October 2018 |
| | | | | AU | 2016357728 | B2 | 27 August 2020 |
| | | | | KR | 20170130615 | A | 28 November 2017 |
| | | | | WO | 2017087428 | A1 | 26 May 2017 |
| | | | | JP | 2018517710 | A | 05 July 2018 |
| | | | | TW | I702222 | B | 21 August 2020 |
| | | | | JP | 6695360 | B2 | 20 May 2020 |
| | | | | HK | 1250988 | A1 | 18 January 2019 |
| | | | | EP | 3390415 | B1 | 26 February 2020 |
| | | | | AU | 2016357728 | A1 | 30 November 2017 |
| | | | | US | 10409869 | B2 | 10 September 2019 |
| | | | | US | 2018258116 | A1 | 13 September 2018 |
| | | | | SG | 11201709123 | A1 | 28 December 2017 |
| | | | | IN | 201817016988 | A | 10 August 2018 |
| CN | 102836433 | A | 26 December 2012 | WO | 2012175052 | A1 | 27 December 2012 |
| CN | 109833321 | A | 04 June 2019 | | None | | |
| WO | 2010044686 | A1 | 22 April 2010 | | None | | |
| WO | 2018148721 | A1 | 16 August 2018 | | None | | |
| WO | 2019002015 | A1 | 03 January 2019 | EP | 3421483 | A1 | 02 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016021581 W
- WO 2016145092 A
- CN 2016800150788
- CN 107530556 A
- US 2016062114 W
- WO 2017087428 A1
- CN 201680044608 1
- CN 108290911 A
- US 2016025665 W
- WO 2016161342 A
- CN 2016800200132
- CN 108136214 A
- US 20130116277 A1
- US 13607633 B
- US 20180305305 A1
- US 15769565 B
- US 20160159731 A1
- US 14993742 B
- US 20140107085 A1
- US 14050937 B
- US 20170260226 A1
- US 15510348 B
- US 20160303082 A1
- US 15132937 B
- US 20180271835 A1
- US 15899171 B
- US 20140371261 A1
- US 14352421 B
- US 20140249119 A1
- US 14348645 B
- US 20150210734 A1
- US 14414386 B
- US 20160024142 A1
- US 14770444 B
- US 20090275608 A1
- US 12322551 B
- US 20170342082 A1
- US 15596383 B
- US 20180319807 A2
- CN 201811133143
- CN 109305972 A
- WO 2016145092 A1
- WO 2017087428 A
- WO 2019190331 A
- CN 201980023423
- CN 111918864 A
- WO 2016061342 A


### Non-patent literature cited in the description


- **KATHRYN EVANS ; JIANXINDUAN ; TARA PRITCHARD.** OBI-3424,a novel AKR1C3-activated prodrug,exhibits potent efficacy against preclinical models of T-ALL[J. *ClinicalCancerResearch,* 2019
- **RICHARD B.LOCK ; KATHRYN EVANS ; RAYMOND YUNG et al.** Abstract LB-B16:The AKR1C3-Activated Prodrug OBI-3424 Exerts Profound InVivo Efficacy Against Preclinical Models of T-Cell Acute Lymphoblastic Leukemia(T-ALL). *Pediatric Preclinical Testing Consortium Study[C], AACR-NCI-EORTC International Conference:Molecular Targetsand Cancer Therapeutics,* 26 October 2017
- **JIANXIN DUAN ; ZHONG WANG ; QING LI et al.** Broad In Vitro and In Vivo Antitumor Activity of TH-3424: Preclinical Rationale for a Highly Selective AKR1C3 Prodrug for Treating Cancer. *AACR Annual Meeting 2016, Abstract #1369,* 16 April 2016
- *CHEMICAL ABSTRACTS,* 2097713-69-2
- **CHRISTOPHER P. GUISE ; MARIA R. ABBATTISTA ; RACHELLE S. SINGLETON et al.** The Bioreductive Prodrug PR-104A Is Activated under Aerobic Conditions by Human Aldo-Keto Reductase 1C3. *Cancer Res,* vol. 70 (4), 1573-1584
- **SAMAD T A ; MOORE K A ; SAPIRSTEIN A et al.** Interleukin-1[beta]-mediated induction of Cox-2 in the CNS contributes to inflammatory pain hypersensitivity[J. *Nature,* 2001, vol. 410 (6827), 471-5

- **BAOJIAN Z ; YANBING Y ; GELE A et al.** Tanshinone IIA Attenuates Diabetic Peripheral Neuropathic Pain in Experimental Rats via Inhibiting Inflammation[J. *Evidence-Based Complementary and Alternative Medicine,* 2018, vol. 2018, 1-8
- **LOVERING A ; RIDE J ; BUNCE C et al.** Crystal Structures of Prostaglandin D2 11-Ketoreductase (AKR1C3) in Complex with the Nonsteroidal Anti-Inflammatory Drugs Flufenamic Acid and Indomethacin[J. *Cancer Research,* 2004, vol. 64 (5), 1802-1810
- **MATSUURA K ; SHIRAISHI H ; HARA A et al.** Identification of a principal mRNA species for human 3alpha-hydroxysteroid dehydrogenase isoform (AKR1C3) that exhibits high prostaglandin D2 11-ketoreductase activity.[J. *Journal of Biochemistry,* 1998, vol. 124 (5), 940-6
- **NOGRADY.** Medicinal Chemistry A Biochemical Approach. Oxford University Press, 1985, 388-392
- **HIGAKI, Y, USAMI et al.** Selective and potent inhibitors of human 20a-hydroxysteroid dehydrogenase (AKR1C1) that metabolizes neurosteroids derived from progesterone[J. *CHEMICOBIOLOGICAL INTERACTIONS,* 2003, 503-513
- **BYDAL P ; LUU-THE V ; LABRIE F et al.** Steroidal lactones as inhibitors of 17beta-hydroxysteroid dehydrogenase type 5: chemical synthesis, enzyme inhibitory activity, and assessment of estrogenic and androgenic activities.[J. *European Journal of Medicinal Chemistry,* 2009, vol. 44 (2), 632-644
- **SKARYDOVA L ; WSOL V ; ZIVNA L et al.** AKR1C3 as a potential target for the inhibitory effect of dietary flavonoids[J. *Chemico-biological interactions,* 2010, vol. 178, 138-144
- **BYRNS M C ; STECKELBROECK S ; PENNING T M.** An indomethacin analogue, N-(4-chlorobenzoyl)-melatonin, is a selective inhibitor of aldo-keto reductase 1C3 (type 2 3alpha-HSD, type 5 17beta-HSD, and prostaglandin F synthase), a potential target for the treatment of hormone dependent and hormone independent malignancies.[J. *Biochemical Pharmacology,* 2008, vol. 75 (2), 484-493
- **BAUMAN, D. R.** Development of nonsteroidal anti-inflammatory drug analogs and steroid carboxylates selective for human aldo-keto reductase isoforms: potential antineoplastic agents that work independently of cyclooxygenase isozymes.[J. *Molecular Pharmacology,* 2005, vol. 67 (1), 60-68
- **PENNING, T, M et al.** Inhibition of a major NAD(P)-linked oxidoreductase from rat liver cytosol by steroidal and nonsteroidal anti-inflammatory agents and by prostaglandins.[J. *Proceedings of the National Academy of Sciences,* 1983, vol. 8, 4504-4508
- **DAVIES N J ; HAYDEN R E ; SIMPSON P J et al.** AKR1C isoforms represent a novel cellular target for jasmonates alongside their mitochondrial-mediated effects.[J. *Cancer Research,* 2009, vol. 69 (11), 4769-75
- **BROZIC P ; GOLOB B ; GOMBOC N et al.** Cinnamic acids as new inhibitors of 17beta-hydroxysteroid dehydrogenase type 5 (AKR1C3).[J. *Molecular & Cellular Endocrinology,* 2006, vol. 248 (1-2), 233-235
- **YINING ZHAO ; XUEHUA ZHENG ; HONG ZHANG et al.** Invitro inhibition of AKRICs by sulphonylureas and the structural basis.[J. *Chemico-Biological Interactions,* 2015, vol. 240, 310-315